# EUROPEAN PATENT APPLICATION

(11) **EP 4 223 881 A1**
(43) Date of publication of application: **09.08.2023**
(21) Application number: 21874539.6
(22) Date of filing: 29.09.2021
(51) Int. Cl.: C12N 15/85, C12N 15/66, C12N 15/64, C12N 15/10

(54) **NUCLEIC ACID DELIVERY METHOD AND SYSTEM**

(30) Priority: 30.09.2020 CN 202011058515
(71) Applicant: Shanghai Ranai Marketing Consulting Partnership (Limited Partnership), Shanghai 201607 (CN)
(72) Inventor: SONG, Jie, Shanghai 200240 (CN); CHENG, Jin, Shanghai 200240 (CN); TANG, Linlin, Shanghai 200240 (CN)
(74) Representative: Sun, Yiming
(86) International application number: PCT/CN2021/121765
(87) International publication number: WO 2022/068884

(57) **Abstract**

The present application relates to a method and system for delivering a nucleic acid. In particular, the present application relates to a method and system for expressing exogenous RNA and/or exogenous proteins by nucleic acid delivery. The present application provides a composition, including single-stranded DNA and at least one transfection reagent. A mass ratio of the single-stranded DNA to the transfection reagent in the composition is about 100 to about 0.1. The present invention further provides a method and system for delivering a nucleic acid using the composition.

## Description

### TECHNICAL FIELD

The present application relates to a method and system for delivering nucleic acid. In particular, the present application relates to a method and system for expressing exogenous RNA and/or exogenous protein by nucleic acid delivery.

### DESCRIPTION OF RELATED ART

Traditional gene delivery refers to a process of introducing nucleic acid molecules such as plasmid DNA (pIDNA) or RNA into cells or organisms by using delivery vectors or specific technologies.

However, the use of double-stranded pIDNA to deliver nucleic acids often introduces sequences from a host, resulting in the expression of non-target proteins. In addition, the expression efficiency of exogenous genes is unsatisfactory, especially for long nucleic acid molecules. In a further aspect, RNA is difficult to obtain, unstable and easy to degrade. Thus, it is not satisfactory for delivering and expressing exogenous genes. Therefore, it is urgent to create a more safe and efficient delivery method of exogenous nucleic acids.

### SUMMARY

The present application provides a method and system for delivering a nucleic acid. The inventor of the present application is surprised to find that the method and system of the present application are used to deliver exogenous nucleic acid molecules to obtain one or more of the following advantages (e.g., compared with the traditional double-stranded plasmid DNA delivery system): 1) higher delivery efficiency (e.g., higher cell transfection efficiency) and higher expression; 2) the capability of effectively delivering longer nucleic acid molecules to express; 3) the capability of reducing the cytotoxicity related to DNA transfection; 4) and/or the capability of reducing the immune response related to DNA transfection, thereby increasing the safety.

In one aspect, the present application provides a composition (e.g., a transfection composition). The composition may include: single-stranded DNA and at least one transfection reagent. In the composition, a mass ratio of the single-stranded

DNA to the transfection reagent may be about 100 to about 0.1.

In certain embodiments, the mass ratio of the single-stranded DNA to the transfection reagent in the composition is about 100:1 to about 1:10.

In certain embodiments, the mass ratio of the single-stranded DNA to the transfection reagent in the composition is about 1 to about 0.2.

In certain embodiments, the mass ratio of the single-stranded DNA to the transfection reagent in the composition is about 2:1 to about 1:5.

In certain embodiments, the single-stranded DNA is circular single-stranded DNA.

In certain embodiments, the single-stranded DNA is linear single-stranded DNA.

In certain embodiments, the single-stranded DNA includes a nucleic acid sequence coding one or more RNAs and/or proteins.

In certain embodiments, the single-stranded DNA includes a nucleic acid sequence coding two or more RNAs and/or proteins.

In certain embodiments, the single-stranded DNA includes a sense strand nucleic acid sequence coding RNA and/or protein.

In certain embodiments, the single-stranded DNA includes an antisense strand nucleic acid sequence coding RNA and/or protein.

In certain embodiments, the single-stranded DNA includes at least 40 nucleotides.

In certain embodiments, the single-stranded DNA includes at least 1500 nucleotides.

In certain embodiments, the transfection reagent includes a cationic lipid and/or cationic polymer.

In certain embodiments, the transfection reagent includes a cationic liposome.

In certain embodiments, the composition also includes one or more cell cultures.

In certain embodiments, the content of the single-stranded DNA in the composition is about 10ng to about 1000ng.

In certain embodiments, a concentration of the single-stranded DNA in the composition is from about 0.5nM to about 50nM.

In a further aspect, the present application provides a kit, which may include the composition (e.g., the transfection composition) according to the present application.

In a further aspect, the present application provides a kit. The kit may include: single-stranded DNA, at least one transfection reagent, and instructions for use (such as specifications for use). The instructions for use may record that the single-stranded DNA and the transfection reagent are applied to target cells in a mass ratio of about 100 to about 0.1 to deliver at least part of the nucleic acid contained in the single-stranded DNA to the target cells.

In certain embodiments, the instructions for use record that the single-stranded DNA and the transfection reagent are applied to the target cells in a mass ratio of about 5:1 to about 1:10 to deliver at least part of the nucleic acid contained in the single-stranded DNA to the target cells.

In certain embodiments, the instructions for use record that the single-stranded DNA and the transfection reagent are applied to the target cells in a mass ratio of about 10 to about 0.1 to deliver at least part of the nucleic acid contained in the single-stranded DNA to the target cells.

In certain embodiments, the instructions for use record that the single-stranded DNA and the transfection reagent are applied to the target cells in a mass ratio of about 2:1 to about 1:5 to deliver at least part of the nucleic acid contained in the single-stranded DNA to the target cells.

In certain embodiments, the instructions for use record that the single-stranded DNA and the transfection reagent are mixed according to said mass ratio to prepare a transfection composition.

In certain embodiments, the single-stranded DNA and the at least one transfection reagent are included in the kit in a manner of not mixing with each other.

In certain embodiments, in the kit, the single-stranded DNA and at least one transfection reagent are separately stored in a separately packaged container.

In certain embodiments, the content of the single-stranded DNA in the kit is about 10ng to about 1000ng.

In certain embodiments, a concentration of the single-stranded DNA in the kit is from about 0.5nM to about 50nM.

In certain embodiments, the single-stranded DNA is circular single-stranded DNA.

In certain embodiments, the single-stranded DNA is linear single-stranded DNA.

In certain embodiments, the single-stranded DNA includes a nucleic acid sequence coding one or more RNAs and/or proteins.

In certain embodiments, the single-stranded DNA includes a nucleic acid sequence coding two or more RNAs and/or proteins.

In certain embodiments, the single-stranded DNA includes a sense strand nucleic acid coding RNA and/or protein.

In certain embodiments, the single-stranded DNA includes an antisense strand nucleic acid sequence coding RNA and/or protein.

In certain embodiments, the single-stranded DNA in the kit contains at least 40 nucleotides.

In certain embodiments, the single-stranded DNA in the kit contains at least 1500 nucleotides.

In certain embodiments, the transfection reagent includes a cationic lipid and/or cationic polymer.

In certain embodiments, the transfection reagent in the kit includes a cationic liposome.

In certain embodiments, the kit also includes one or more cell culture mediums.

In a further aspect, the present application provides a method for delivering a nucleic acid to cells. The method may include: contacting the cells with the single-stranded DNA containing the nucleic acid and the transfection reagent. A mass ratio of the single-stranded DNA to the transfection reagent may be about 5 to about 0.1.

In certain embodiments of the method, the mass ratio of the single-stranded DNA to the transfection reagent is about 5:1 to about 1:10.

In certain embodiments of the method, the mass ratio of the single-stranded DNA to the transfection reagent is about 1 to about 0.2.

In certain embodiments of the method, the mass ratio of the single-stranded DNA to the transfection reagent is about 2:1 to about 1:5.

In a further aspect, the present application provides a method for expressing exogenous RNA and/or exogenous protein in cells. The method may includes: introducing single-stranded DNA coding the exogenous RNA and/or the exogenous protein into the cells.

In certain embodiments of the method, the method also includes: culturing the cells under a condition that allows the expression of the exogenous RNA and/or the exogenous protein.

In a further aspect, the present application provides a method for reducing the cytotoxicity related to DNA transfection. The method may include: introducing single-stranded DNA rather than corresponding double-stranded DNA into the cells during transfection.

In a further aspect, the present application provides a method for reducing the cell immunogenicity related to DNA transfection. The method may include: introducing single-stranded DNA rather than corresponding double-stranded DNA into the cells during transfection.

In a further aspect, the present application provides a method for improving the efficiency of DNA transfection of cells. The method may include: introducing single-stranded DNA rather than corresponding double-stranded DNA into the cells during transfection.

In certain embodiments of the method in the present application, the step of introducing the single-stranded DNA into the cells includes: contacting the cells with the composition in the present application.

In certain embodiments of the method in the present application, the step of introducing the single-stranded DNA into the cells includes: contacting the cells with the single-stranded DNA and the transfection reagent, wherein a mass ratio of the single-stranded DNA to the transfection reagent is about 5 to about 0.1.

In certain embodiments of the method in the present application, the step of introducing the single-stranded DNA into the cells includess: contacting the cells with the single-stranded DNA and the transfection reagent, wherein the mass ratio of the single-stranded DNA to the transfection reagent is about 5:1 to about 1:10.

In certain embodiments of the method in the present application, the step of introducing the single-stranded DNA into the cells includes: contacting the cells with the single-stranded DNA and the transfection reagent, wherein the mass ratio of the single-stranded DNA to the transfection reagent is about 1 to about 0.2.

In certain embodiments of the method in the present application, the step of introducing the single-stranded DNA into the cells includes: contacting the cells with the single-stranded DNA and the transfection reagent, wherein the mass ratio of the single-stranded DNA to the transfection reagent is about 2:1 to about 1:5.

In certain embodiments of the method in the present application, the contact (e.g., contacting the cells with the single-stranded DNA and the transfection reagent, or contacting the cells with the composition in the present application) includes: incubating at about 25°C to about 40°C.

In certain embodiments of the method in the present application, the contact includes: incubating at about 37°C.

In certain embodiments of the method in the present application, the contact includes: incubating for at least 4 hours.

In certain embodiments of the method in the present application, the single-stranded DNA is circular single-stranded DNA.

In certain embodiments of the method in the present application, the single-stranded DNA is linear single-stranded DNA.

In certain embodiments of the method in the present application, the single-stranded DNA includes a nucleic acid sequence coding one or more exogenous RNAs and/or exogenous proteins.

In certain embodiments of the method in the present application, the single-stranded DNA includes a nucleic acid sequence coding two or more exogenous RNAs and/or exogenous proteins.

In certain embodiments of the method in the present application, the single-stranded DNA includes a sense strand nucleic acid sequence coding exogenous RNA and/or exogenous protein.

In certain embodiments of the method in the present application, the single-stranded DNA includes an antisense strand nucleic acid sequence coding exogenous RNA and/or exogenous protein.

In certain embodiments of the method in the present application, the single-stranded DNA includes at least 40 nucleotides.

In certain embodiments of the method in the present application, the single-stranded DNA includes at least 1500 nucleotides.

In certain embodiments of the method in the present application, the content of the single-stranded DNA is about 10ng to about 1000ng.

In certain embodiments of the method in the present application, a concentration of the single-stranded DNA is about 0.5nM to about 50nM.

In certain embodiments of the method in the present application, the transfection reagent includes a cationic lipid and/or cationic polymer.

In certain embodiments of the method in the present application, the transfection reagent includes a cationic liposome.

In certain embodiments, the method in the present application is an *in vivo* method or *in vitro* method.

In certain embodiments of the method in the present application, the cells are somatic cells, tumor cells and/or immune cells.

In certain embodiments of the method in the present application, the cells are selected from: lung cancer cells, kidney cells, melanoma cells, liver cancer cells, cervical cancer cells, glioma cells, epithelial cells, colon cancer cells, fibroblasts, breast cancer cells, stomach cancer cells, and immune effector cells.

In certain embodiments of the method in the present application, the cells are selected from: A549 cells, C6 cells, MCF-7 cells, HepG2 cells, CT26 cells, Hela cells, Helf cells, MDCK cells, B16 cells, Hep3B cells, L929 cells, 4T1 cells, 293T cells, MGC803 cells and macrophages.

In a further aspect, the present application provides cells obtained by processing with the method in any aspect of the present application.

In a further aspect, the present application provides an organism (e.g., non-human organism) or components thereof, which may include the cells in the present application (e.g., cells processed with the method in the present application).

In certain embodiments, the components are selected from tissues, organs and combinations thereof.

In a further aspect, the present application provides use of single-stranded DNA in the preparation of a composition (such as a transfection composition, e.g., a cell transfection composition, or a nucleic acid delivery composition). The composition may be used for one or more of the followings: 1) delivering at least part of nucleic acid contained in the single-stranded DNA to cells; 2) expressing exogenous RNA or exogenous protein encoded by the single-stranded DNA in the cells; 3) improving the DNA transfection efficiency of the cells; 4) reducing the cytotoxicity related to DNA transfection; and/or 5) reducing the cellular immunogenicity related to DNA transfection. The composition may be the composition in any aspect of the present application.

In certain embodiments, the cells are somatic cells, tumor cells, and/or immune cells.

In certain embodiments, the cells are selected from: lung cancer cells, kidney cells, melanoma cells, liver cancer cells, cervical cancer cells, glioma cells, epithelial cells, colon cancer cells, fibroblasts, breast cancer cells, stomach cancer cells, and immune effector cells.

In certain embodiments, the cells are selected from: A549 cells, C6 cells, MCF-7 cells, HepG2 cells, CT26 cells, Hela cells, Helf cells, MDCK cells, B16 cells, Hep3B cells, L929 cells, 4T1 cells, 293T cells, MGC803 cells, and macrophages.

Those skilled in the art can easily recognize other aspects and advantages of the present application from the following detailed description. Only exemplary embodiments of the present application are shown and described in the following detailed description. As those skilled in the art will realize, the content of the present application enables those skilled in the art to modify the disclosed specific embodiments without departing from the spirit and scope of the invention involved in the present application. Accordingly, the description in the drawings and description of the present application is only illustrative, but not restrictive.

### BRIEF DESCRIPTION OF THE DRAWINGS

In order to more clearly illustrate the embodiments of the present invention or the technical solutions in the prior art, the accompanying diagrams used in the embodiments will be described briefly:
FIG. 1 shows structures and construction processes of exemplary vectors pScaf-EGFP (+), pScaf-EGFP (-) and pScaf-EGFP-mCherry in the present application;
FIGs. 2A-2B show the transfection efficiency and protein expression of the exemplary single-stranded DNA of the present application in each cell line;
FIG. 3 shows the protein expression of the exemplary single-stranded DNA of the present application in each cell line;
FIGs. 4A-4C show the protein expression of MDCK cells transfected with exemplary single-stranded DNA of the present application;
FIG. 5 shows the protein expression of the transfected cells of the exemplary single-stranded DNA of the present application compared to the corresponding double-stranded plasmid DNA;
FIG. 6 shows the protein expression of the transfected cells of the exemplary single-stranded DNA of the present application compared to the corresponding double stranded plasmid DNA;
FIG. 7 shows the protein expression of the transfected cells of the exemplary single-stranded DNA of the present application compared to the corresponding double stranded plasmid DNA;
FIG. 8 shows a cytotoxic effect of the exemplary single-stranded DNA of the present application after transfection into cells compared to the corresponding double-stranded plasmid DNA;
FIG. 9 shows an electrophoretic verification result of the exemplary linear single-stranded DNA in the present application, in which1kb shows a 1 kb DNA marker, a first lane is the corresponding double-stranded DNA, a second lane is a reverse product amplification result, and a third lane is a forward product amplification result;
FIG. 10 shows the expression of the exemplary linear single-stranded DNA in the present application after transfection into different cell lines;
FIG. 11 shows a DNA enzyme (S1 enzyme) verification result of the exemplary single-stranded DNA in the present application;
FIGs. 12A-12B show the effects of the exemplary single-stranded DNA in the present application in inducing immune response compared to the double-stranded plasmid DNA; and
FIGs. 13A-13B show the effects of the exemplary single-stranded DNA in the present application in inducing immune response compared to the double-stranded plasmid DNA.

### DESCRIPTION OF THE EMBODIMENTS

The invention involved in the present application is described in the following specific embodiments. Those familiar with this technology can easily understand other advantages and effects of the invention of the present application from the content disclosed in this description.

In the present application, the terms "nucleic acid", "nucleic acid molecule" and "polynucleotide" are used interchangeably, usually referring to the combination of at least two base-sugar-phosphate units. Here, the difference between polynucleotide and oligonucleotide is that the polynucleotide usually contains more than 120 monomer units.

In the present application, the "nucleic acid" includes deoxyribonucleic acid (DNA) and ribonucleic acid (RNA). Nucleotides are monomer units of nucleic acid polymers. Antisense nucleic acids are nucleic acids that interfere with DNA and/or RNA functions. Natural nucleic acids have phosphate skeletons, while artificial nucleic acids may contain other types of skeletons, but have the same base as the natural nucleic acids. RNA may be tRNA (transfer RNA), snRNA (small nuclear RNA), rRNA (ribosomal RNA), mRNA (messenger RNA), antisense RNA and/or ribozyme. DNA may be plasmid DNA, viral DNA, linear DNA, chromosome DNA or their derivatives. In addition to these forms, DNA and RNA may also be single-stranded, double-stranded, triple-stranded or quad-stranded forms. The term "nucleic acid" may also include variations of peptide nucleic acid (PNA), thiophosphate and other natural nucleic acid phosphate skeletons.

In the present application, the term "single-stranded DNA" is also referred to as "ssDNA", which usually refers to a nucleic acid molecule mainly composed of one DNA strand. DNA can exist in a double-stranded or single-stranded form. In double-stranded DNA (dsDNA), two reverse parallel DNA strands bind to each other through Watson-Crick binding complementation (guanine-cytosine or adenine-thymine pairing) between guanine-cytosine and adenine-thymidine pairs, for example. On the contrary, single-stranded (ssDNA) DNA usually has only one DNA strand. In this DNA strand, there are at least two base-sugar-phosphate combinations, which are covalently bound to each other. In some cases, single-stranded DNA can form a loop or secondary structure within itself, and Watson-Crick pairing occurs between two or more different parts of one strand. ssDNA may also include a portion of which has a single-stranded structure, in which DNA may have double-stranded and single-stranded segments. For example, ssDNA may have a portion that is not paired with another DNA strand and the other portion that is paired with another DNA strand. ssDNA may be formed in many ways. For example, ssDNA may be formed by denaturing dsDNA. Denaturing refers to a process of separating two strands of dsDNA to reduce the number of Watson-Crick pairs. ssDNA may also be formed by nucleic acid amplification (e.g., rolling circle amplification or asymmetric amplification). The ssDNA may be circular or linear.

In the present application, the terms "exogenous DNA sequence", "exogenous nucleic acid" and "exogenous DNA" are used interchangeably, usually referring to nucleic acid molecules that do not originate from a host in which they are located. It may be the same as or heterologous to the host's DNA. For example, a exogenous DNA sequence may be a nucleic acid sequence inserted into an expression vector to express target RNA or protein (e.g., exogenous RNA or exogenous protein). The exogenous DNA sequence may be derived from various sources, including DNA, cDNA, synthetic DNA, RNA, etc. The exogenous DNA sequence may contain genomic DNA, and the genomic DNA may or may not include natural or artificial introns. In addition, the genomic DNA may be connected with a promoter region or polyA signal sequence. The exogenous DNA sequence includes, but is not limited to, the following DNA sequences, which can express gene products (such as target RNA or target protein) that are expected to be expressed in a host cell. The gene product can affect the activity or state of host cells.

The term "about" or "approximately" usually denotes a statistically significant range of values. Such a range may be within an order of magnitude, for example, within 50%, 20%, 15%, 10%, or 5% above and below a given value or range. The allowable deviation covered by the term "about" or "approximately" depends on the specific system under study, which may be understood by those skilled in the art according to the content of the present application.

### COMPOSITION:

In one aspect, the present application provides a composition (e.g., a transfection composition). The composition may include: single-stranded DNA and at least one transfection reagent. In the composition, a mass ratio of the single-stranded DNA to the transfection reagent may range from about 5 to about 0.1. For example, the mass ratio of the single-stranded DNA to the transfection reagent may be about 4.5 to about 0.5, about 4 to about 1, about 3.5 to about 1.5, about 3 to about 0.5, about 2.5 to about 0.1, about 2 to about 0.1, about 1.5 to about 0.1, about 1 to about 0.1, about 1 to about 0.2, about 1.5 to about 0.25, about 1.5 to about 0.3, about 1 to about 0.25, about 1.25 to about 0.2, about 0.75 to about 0.25, about 0.5 to about 0.3, etc. For example, the mass ratio of the single-stranded DNA to the transfection reagent may be about 100 to about 0.1, about 50 to about 0.1, about 20 to about 0.1, about 10 to about 0.1, about 5 to about 0.1, about 100 to about 0.5, about 50 to about 0.5, about 20 to about 0.5, about 10 to about 0.5, about 5 to about 0.5, about 100 to about 1, about 50 to about 1, about 20 to about 1, about 10 to about 1, or about 5 to about 1, etc.

In certain embodiments, the mass ratio of the single-stranded DNA to the transfection reagent in the composition is about 5:1 to about 1:10. About 5:1 to about 1:10 means that the mass of the single-stranded DNA relative to the mass of the transfection reagent gradually decreases from about 5:1 until they are basically the same (that is, the mass ratio of the two is about 1:1), and then the relative mass of the transfection reagent continues to increase until the mass of the single-stranded DNA relative to the mass of the transfection reagent is about 1:10. In certain cases, the mass ratio of the single-stranded DNA to the transfection reagent in the composition is about 4:1 to about 1:10, about 3:1 to about 1:10, about 2:1 to about 1:10, about 1:1 to about 1:10, about 1:1 to about 1:9, about 1:1 to about 1:8, about 1:1 to about 1:7, about 1:1 to about 1:6, about 1:1 to about 1:5, about 1:1 to about 1:4, about 1:1 to about 1:3, about 1:1 to about 1:2, about 2:1 to about 1:2, about 2:1 to about 1:2, about 2:1 to about 1:3, about 2:1 to about 1:4, about 2:1 to about 1:5, etc. For example, this mass ratio may be about 100:1 to about 1:10, about 100:1 to about 1:10, about 50:1 to about 1:10, about 20:1 to about 1:10, about 10:1 to about 1:10, about 100:1 to about 1:5, about 100:1 to about 1:5, about 50:1 to about 1:5, about 20:1 to about 1:5, about 10:1 to about 1:5, about 100:1 to about 1:1, about 100:1 to about 1:1, about 50:1 to about 1:1, about 20:1 to about 1:1, or about 10:1 to about 1:1, etc.

In certain embodiments, the single-stranded DNA in the composition is circular single-stranded DNA, and a mass ratio of the circular single-stranded DNA to the transfection reagent is about 5:1 to about 1:10. For example, this mass ratio may be about 4:1 to about 1:10, about 3:1 to about 1:10, about 2:1 to about 1:10, about 1:1 to about 1:10, about 1:1 to about 1:9, about 1:1 to about 1:8, about 1:1 to about 1:7, about 1:1 to about 1:6, about 1:1 to about 1:5, about 1:1 to about 1:4, about 1:1 to about 1:3, about 1:1 to about 1:2, about 2:1 to about 1:2, about 2:1 to about 1:3, about 2:1 to about 1:3, about 2:1 to about 1:4, about 2:1 to about 1:5, etc. For example, this mass ratio may be about 100:1 to about 1:10, about 50:1 to about 1:10, about 20:1 to about 1:10, about 10:1 to about 1:10, about 100:1 to about 1:5, about 100:1 to about 1:5, about 50:1 to about 1:5, about 20:1 to about 1:5, about 10:1 to about 1:5, about 100:1 to about 1:1, about 100:1 to about 1:1, about 50:1 to about 1:1, about 20:1 to about 1:1, or about 10:1 to about 1:1.

In certain embodiments, the single-stranded DNA in the composition is linear single-stranded DNA, and a mass ratio of the linear single-stranded DNA to the transfection reagent is about 5:1 to about 1:10. For example, this mass ratio may be about 4:1 to about 1:10, about 3:1 to about 1:10, about 2:1 to about 1:10, about 1:1 to about 1:10, about 1:1 to about 1:9, about 1:1 to about 1:8, about 1:1 to about 1:7, about 1:1 to about 1:6, about 1:1 to about 1:5, about 1:1 to about 1:4, about 1:1 to about 1:3, about 1:1 to about 1:2, about 2:1 to about 1:2, about 2:1 to about 1:3, about 2:1 to about 1:4, about 2:1 to about 1:5, etc. For example, this mass ratio may be about 100:1 to about 1:10, about 100:1 to about 1:10, about 50:1 to about 1:10, about 20:1 to about 1:10, about 10:1 to about 1:10, about 100:1 to about 1:5, about 50:1 to about 1:5, about 20:1 to about 1:5, about 10:1 to about 1:5, about 100:1 to about 1:1, about 100:1 to about 1:1, about 50:1 to about 1:1, about 20:1 to about 1:1, or about 10:1 to about 1:1, etc.

In certain embodiments, the single-stranded DNA includes a nucleic acid coding one or more RNAs and/or proteins. For example, the single-stranded DNA may be transcribed/reversely transcribed into RNA molecules (e.g., miRNA, mRNA or other RNA molecules) in cells as templates. In certain cases, the single-stranded DNA may be transformed into double-stranded DNA, which can exist in a cell freely, and may also be integrated into a genome (e.g., in a random insertion mode, or integrated into a specific site) of the cell. The single-stranded DNA may be transcribed and translated to express a target protein it encodes (e.g., a therapeutic protein, a marker protein such as a fluorescent protein or a luminescent protein, etc.).

In certain embodiments, the single-stranded DNA includes a nucleic acid sequence coding two or more RNAs and/or proteins. For example, the single-stranded DNA may contain a first coding region and a second coding region, wherein the first coding region may encode a first RNA or a first protein, and the second coding region may independently encode a second RNA or a second protein that is the same as or different from the first RNA or the first protein. The first coding region and the second coding region may contain one or more non-coding regions. In certain cases, there is basically no non-coding region between the first coding region and the second coding region. In certain cases, the single-stranded DNA may contain a third coding region or more coding regions. Expression products of each coding region may form protein complexes or protein/nucleic acid complexes.

For example, the single-stranded DNA (e.g., single-stranded circular DNA or single-stranded linear DNA) may include a nucleic acid sequence coding one or more RNAs and/or proteins (e.g., a nucleic acid sequence coding two or more RNAs and/or proteins), which may include at least about 40 nucleotides, for example, at least about 50 nucleotides, at least about 100 nucleotides, at least about 200 nucleotides, at least about 300 nucleotides, and at least about 400 nucleotides, at least 450 nucleotides, at least 500 nucleotides, at least 550 nucleotides, at least 600 nucleotides, at least 650 nucleotides, at least 700 nucleotides, at least 750 nucleotides, at least 800 nucleotides, at least 850 nucleotides, at least 900 nucleotides, at least 950 nucleotides, at least 1000 nucleotides, at least 1050 nucleotides, at least 1100 nucleotides, at least 1150 nucleotides, at least 1200 nucleotides, at least 1250 nucleotides, at least 1300 nucleotides, at least 1350 nucleotides, at least 1400 nucleotides, at least 1450 nucleotides, at least 1500 nucleotides, at least 1550 nucleotides, at least 1600 nucleotides, at least 1650 nucleotides, at least 1700 nucleotides, at least 1750 nucleotides, at least 1800 nucleotides, at least 1850 nucleotides, at least 1900 nucleotides, at least 1950 nucleotides, at least 2000 nucleotides, at least 2100 nucleotides, at least 2200 nucleotides, at least 2300 nucleotides, at least 2400 nucleotides, at least 2500 nucleotides, at least 2600 nucleotides, at least 2700 nucleotides, at least 2800 nucleotides, at least 2900 nucleotides, at least 3000 nucleotides, at least 3100 nucleotides, at least 3200 nucleotides, at least 3300 nucleotides, at least 3400 nucleotides, at least 3500 nucleotides, at least 3600 nucleotides, at least 3700 nucleotides, at least 3800 nucleotides, at least 3900 nucleotides, at least 4000 nucleotides, at least 4100 nucleotides, at least 4200 nucleotides, at least 4300 nucleotides, at least 4400 nucleotides, at least 4500 nucleotides, at least 4600 nucleotides, at least 4700 nucleotides, at least 4800 nucleotides, at least 4900 nucleotides, at least 5000 nucleotides, at least 5100 nucleotides, at least 5200 nucleotides, at least 5300 nucleotides, at least 5400 nucleotides, at least 5500 nucleotides, at least 5600 nucleotides, at least 5700 nucleotides, at least 5800 nucleotides, at least about 5900 nucleotides, at least about 6000 nucleotides or more.

In certain embodiments, the single-stranded DNA includes a sense strand (also known as a coding strand, a sense strand, a positive strand, nontemplete strand or a +strand) nucleic acid sequence coding RNA and/or protein. For example, the nucleic acid sequence contained in the single-stranded DNA is basically consistent with the coding sequence (e.g., a sequence of mRNA coding the target protein) of the target protein expressed.

In certain embodiments, the single-stranded DNA includes an antisense strand nucleic acid sequence coding RNA and/or protein. For example, the antisense strand nucleic acid sequence and the sense strand sequence (e.g., a coding nucleic acid sequence of the target protein) are basically complementary. In certain embodiments, the antisense strand nucleic acid sequence and the sense strand sequence are completely complementary.

The single-stranded DNA in the present application may also include other elements, such as a promoter sequence (e.g., CMV promoter), an enhancer sequence and/or a polyA sequence (e.g., SV40 polyA).

The composition of the present application also includes at least one transfection reagent.

In the present application, the term "transfection" usually refers to a process or action of transferring a nucleic acid (e.g., exogenous nucleic acid) from the outside of a cell membrane to the inside of the cell membrane, or may also refer to a process of introducing nucleic acid (e.g., exogenous nucleic acid) into cells (e.g., eukaryotic cells, such as mammalian cells). In the present application, "transfection" includes, for example, "stable transfection" and "transient transfection". The "stable transfection" usually refers to the introduction and integration of exogenous nucleic acids (e.g., DNA) into genomes of the transfected cells. The "instantaneous transfection" usually refers to the introduction of exogenous nucleic acids (e.g., DNA) into the cells, but the exogenous nucleic acids are not integrated with the genomes of the transfected cells. Nucleic acid transfection may be carried out in various ways, such as electroporation, use of transfection reagents, etc.

In the present application, the "transfection reagent" may refer to a substance (e.g., a compound) used to mediate nucleic acid to enter into cells. For example, the transfection reagents may include, but are not limited to, cationic liposomes, lipids, polyamines, calcium phosphate, polyimide, polylysine, and/or combinations thereof. The transfection reagent may have a positive charge combined with a negative charge of nucleic acid. For example, cationic liposomes or polylysine complexes have net positive charges, allowing them to bind to DNA or RNA.

For example, the transfection reagent may include a polymer, such as a DNA polycation complex. The transfection reagent may include a cationic protein (e.g., histone or protamine) or a synthetic polymer (e.g., polylysine, polyarginine, DEAE glucan, polyphenylene ether, polyethyleneimine, etc.). In certain embodiments, the transfection reagent includes a cationic lipid (e.g., a cationic liposome, such as Lipofectamine 2000 or Lipofectamine 3000).

Other substances that may be used as the transfection reagents may include, for example, pH sensitive lipids, amphiphilic compounds, other liposomes, etc. In certain embodiments, the transfection reagent may include 1,2-dioleoyl-3-trimethylammonium-propane chloride (DOTMA), dioleoyl phosphatidylethanolamine (DOPE), dimethyl-2,3-dioleoxypropyl-2-(2-arginamido)ethylammonium trifluoroacetate (DOSPA), N-(2-arginine formyl)-N',N'-dioctadecyl glycamide (DOGS) and/or other substances that may form liposomes.

In certain embodiments, the composition also includes one or more cell culture mediums. For example, it may include an MEM medium (a minimum essential medium, a minimum basic medium or a low limit Eagle medium), etc. The content of the medium may be, for example, at least about 20µl, at least about 30µl, at least about 40µl, at least about 50µl, at least about 60µl, at least about 70µl, at least about 80µl, at least about 90µl, at least about 100µl, at least about 110µl, at least about 120µl, at least about 130µl, at least about 140µl, at least about 150µl, at least about 160µl, at least about 170µl, at least about 180µl, at least about 190µl, at least about 200µl, at least about 300µl, at least about 400µl, at least about 500µl, at least about 600µl or more.

In certain embodiments, the content of the single-stranded DNA (e.g., circular single-stranded DNA, or linear single-stranded DNA) in the composition is about 10ng to about 1500ng. For example, this content may be about 10ng to about 1400ng, about 10ng to about 1300ng, about 10ng to about 1200ng, about 10ng to about 1100ng, about 10ng to about 1000ng, about 10ng to about 950ng, about 10ng to about 900ng, about 10ng to about 850ng, about 10ng to about 800ng, about 10ng to about 750ng, about 10ng to about 700ng, about 10ng to about 650ng, about 10ng to about 600ng, about 10ng to about 550ng, about 10ng to about 500ng, about 10ng to about 450ng, about 10ng to about 400ng, about 10ng to about 350ng, About 10ng to about 300ng, about 10ng to about 250ng, about 10ng to about 200ng, about 10ng to about 150ng, about 10ng to about 100ng, about 10ng to about 50ng, etc.

In certain embodiments, a concentration of the single-stranded DNA (e.g., circular single-stranded DNA, or linear single-stranded DNA) in the composition is about 0.1 nM to about 5nM. For example, this concentration may be about 0.2nM to about 4.5nM, about 0.3nM to about 4nM, about 0.4nM to about 3.5nM, about 0.5nM to about 3nM, about 0.6nM to about 2.5nM, about 0.7nM to about 2nM, about 0.8nM to about 1.9nM, about 0.9nM to about 1.8nM, about 1 nM to about 1.6nM, about 1.1 nM to about 1.5nM, about 1.2nM to about 1.4nM, about 1.3nM to about 1.7nM, etc.

### Kit:

In a further aspect, the present application provides a kit, which may include the composition (e.g., a transfection composition) in the present application.

In a further aspect, the present application provides a kit. The kit may include: single-stranded DNA (e.g., the single-stranded DNA in the present application), at least one transfection reagent (e.g., the transfection reagent in the present application), and instructions for use (e.g., a specification for use). The instructions for use can record that the single-stranded DNA and the transfection reagent are applied to target cells in a mass ratio of about 5 to about 0.1 to deliver at least part of the nucleic acid contained in the single-stranded DNA to the target cells. For example, a mass ratio of the single-stranded DNA to the transfection reagent may be about 4.5 to about 0.5, from about 4 to about 1, about 3.5 to about 1.5, about 3 to about 0.5, about 2.5 to about 0.1, about 2 to about 0.1, about 1.5 to about 0.1, about 1 to about 0.1, about 1 to about 0.2, about 1.5 to about 0.25, about 1.5 to about 0.3, about 1 to about 0.25, about 1.25 to about 0.2, about 0.75 to about 0.25, about 0.5 to about 0.3, etc. For example, the mass ratio of the single-stranded DNA to the transfection reagent may be about 100 to about 0.1, about 50 to about 0.1, about 20 to about 0.1, about 10 to about 0.1, about 5 to about 0.1, about 100 to about 0.5, about 50 to about 0.5, about 20 to about 0.5, about 10 to about 0.5, about 5 to about 0.5, about 100 to about 1, about 50 to about 1, about 20 to about 1, about 10 to about 1, or about 5 to about 1.

In certain embodiments, the instructions for use record that the single-stranded DNA and the transfection reagent are applied to the target cells in a mass ratio of about 5:1 to about 1:10 to deliver at least part of the nucleic acid contained in the single-stranded DNA to the target cells. For example, the mass ratio of the single-stranded DNA to the transfection reagent may be about 4:1 to about 1:10, about 3:1 to about 1:10, about 2:1 to about 1:10, about 1:1 to about 1:10, about 1:1 to about 1:9, about 1:1 to about 1:8, about 1:1 to about 1:7, about 1:1 to about 1:6, about 1:1 to about 1:5, about 1:1 to about 1:4, about 1:1 to about 1:3, about 1:1 to about 1:2, about 1:1 to about 1:2, about 2:1 to about 1:2, about 2:1 to about 1:3, about 2:1 to about 1:4, about 2:1 to about 1:5, etc. For example, this mass ratio may be about 100:1 to about 1:10, about 100:1 to about 1:10, about 50:1 to about 1:10, about 20:1 to about 1:10, about 10:1 to about 1:10, about 100:1 to about 1:5, about 100:1 to about 1:5, about 50:1 to about 1:5, about 20:1 to about 1:5, about 10:1 to about 1:5, about 100:1 to about 1:1, about 100:1 to about 1:1, about 50:1 to about 1:1, about 20:1 to about 1:1, or about 10:1 to about 1:1.

In certain embodiments, the instructions for use record that the single-stranded DNA and the transfection reagent are mixed according to said mass ratio to prepare the transfection composition. That is, the single-stranded DNA and the transfection reagent may be first mixed according to said mass ratio described in the present application to obtain the transfection composition (optionally, the transfection composition may also include a buffer or other solvents, such as the cell culture medium in the present application). The transfection composition may then be applied to cells or tissues.

In certain embodiments, the single-stranded DNA and the transfection reagent are directly or simultaneously applied to cells or tissues according to the mass ratio described in the present application, or may be prepared into a transfection composition without pre-mixing.

In certain embodiments, the single-stranded DNA and the at least one transfection reagent are included in the kit in a manner of not mixing with each other. For example, in the kit, the single-stranded DNA and the at least one transfection reagent may be separately stored in a separately packaged container. For example, the kit contains two or more containers independently packaged with each other, each of which contains the single-stranded DNA and the transfection reagent in the present application, and optionally other reagents (e.g., the cell culture medium in the present application).

In the kit, the content of the single-stranded DNA (e.g., circular single-stranded DNA, or linear single-stranded DNA) may be about 10ng to about 1500ng. For example, this content may be about 10ng to about 1400ng, about 10ng to about 1300ng, about 10ng to about 1200ng, about 10ng to about 1100ng, about 10ng to about 1000ng, about 10ng to about 950ng, about 10ng to about 900ng, about 10ng to about 850ng, about 10ng to about 800ng, about 10ng to about 750ng, about 10ng to about 700ng, about 10ng to about 650ng, about 10ng to about 600ng, about 10ng to about 550ng, about 10ng to about 500ng, about 10ng to about 450ng, about 10ng to about 400ng, about 10ng to about 350ng, about 10ng to about 300ng, about 10ng to about 250ng, about 10ng to about 200ng, about 10ng to about 150ng, about 10ng to about 100ng, about 10ng to about 50ng, etc.

In the kit, the concentration of the single-stranded DNA (e.g., circular single-stranded DNA, or linear single-stranded DNA) may be about 0.1nM to about 5nM. For example, this concentration may be about 0.2nM to about 4.5nM, about 0.3nM to about 4nM, about 0.4nM to about 3.5nM, about 0.5nM to about 3nM, about 0.6nM to about 2.5nM, about 0.7nM to about 2nM, about 0.8nM to about 1.9nM, about 0.9nM to about 1.8nM, about 1nM to about 1.6nM, about 1.1nM to about 1.5nM, about 1.2nM to about 1.4nM, about 1.3nM to about 1.7nM, etc.

In the kit, the single-stranded DNA may include a nucleic acid sequence coding one or more RNAs and/or proteins. For example, the single-stranded DNA may be transcribed/reversely transcribed into RNA molecules (e.g., miRNA, mRNA or other RNA molecules) in cells as templates. In some cases, the single-stranded DNA may be transformed into double-stranded DNA, which may exist in the cells freely, and may also be integrated into genomes of the cells (e.g., in a random insertion mode, or integrated into a specific site). The single-stranded DNA may be transcribed and translated to express a target protein (e.g., therapeutic protein, marker protein such as fluorescent protein or luminescent protein).

In the kit, the single-stranded DNA may contain a nucleic acid sequence coding two or more RNAs and/or proteins. For example, the single-stranded DNA may contain a first coding region and a second coding region, wherein the first coding region may encode a first RNA or a first protein, and the second coding region may independently encode a second RNA or a second protein that is the same as or different from the first RNA or the first protein. One or more non-coding regions may be included between the first coding region and the second coding region. In some cases, there is basically no non-coding region between the first coding region and the second coding region. In some cases, the single-stranded DNA may include a third coding region or more coding regions. The expression products of each coding region can form protein complexes or protein/nucleic acid complexes.

For example, the single-stranded DNA (e.g., circular single-stranded DNA or linear single-stranded DNA) may include a nucleic acid sequence coding one or more RNAs and/or proteins (e.g., a nucleic acid sequence coding two or more RNAs and/or proteins), which may contain at least about 40 nucleotides, for example, at least about 50 nucleotides, at least about 100 nucleotides, at least about 200 nucleotides, at least about 300 nucleotides, and at least about 400 nucleotides, at least 450 nucleotides, at least 500 nucleotides, at least 550 nucleotides, at least 600 nucleotides, at least 650 nucleotides, at least 700 nucleotides, at least 750 nucleotides, at least 800 nucleotides, at least 850 nucleotides, at least 900 nucleotides, at least 950 nucleotides, at least 1000 nucleotides, at least 1050 nucleotides, at least 1100 nucleotides, at least 1150 nucleotides, at least 1200 nucleotides, at least 1250 nucleotides, at least 1300 nucleotides, at least 1350 nucleotides, at least 1400 nucleotides, at least 1450 nucleotides, at least 1500 nucleotides, at least 1550 nucleotides, at least 1600 nucleotides, at least 1650 nucleotides, at least 1700 nucleotides, at least 1750 nucleotides, at least 1800 nucleotides, at least 1850 nucleotides, at least 1900 nucleotides, at least 1950 nucleotides, at least 2000 nucleotides, at least 2100 nucleotides, at least 2200 nucleotides, at least 2300 nucleotides, at least 2400 nucleotides, at least 2500 nucleotides, at least 2600 nucleotides, at least 2700 nucleotides, at least 2800 nucleotides, at least 2900 nucleotides, at least 3000 nucleotides, at least 3100 nucleotides, at least 3200 nucleotides, at least 3300 nucleotides, at least 3400 nucleotides, at least 3500 nucleotides, at least 3600 nucleotides, at least 3700 nucleotides, at least 3800 nucleotides, at least 3900 nucleotides, at least 4000 nucleotides, at least 4100 nucleotides, at least 4200 nucleotides, at least 4300 nucleotides, at least 4400 nucleotides, at least 4500 nucleotides, at least 4600 nucleotides, at least 4700 nucleotides, at least 4800 nucleotides, at least 4900 nucleotides, at least 5000 nucleotides, at least 5100 nucleotides, at least 5200 nucleotides, at least 5300 nucleotides, at least 5400 nucleotides, at least 5500 nucleotides, at least 5600 nucleotides, at least 5700 nucleotides, at least 5800 nucleotides, at least about 5900 nucleotides, at least about 6000 nucleotides or more.

In certain embodiments, the single-stranded DNA includes a sense strand (also known as a coding strand, a sense strand, a positive strand, a nontemplete strand or a + strand) nucleic acid sequence coding RNA and/or protein. For example, the nucleic acid sequence contained in the single-stranded DNA is basically consistent with a coding sequence of a target protein expressed (e.g., a sequence of mRNA coding the target protein).

In certain embodiments, the single-stranded DNA includes an antisense strand nucleic acid sequence coding RNA and/or protein. For example, the antisense strand nucleic acid sequence and the sense strand sequence (e.g., a coding nucleic acid sequence of the target protein) are basically complementary. In certain embodiments, the antisense nucleic acid sequence and the sense strand sequence are completely complementary.

The single-stranded DNA described in the present application may also include other elements, such as a promoter sequence (e.g., a CMV promoter), an enhancer sequence and/or a polyA sequence (e.g., SV40 polyA).

The kit may include one or more transfection reagents.

For example, the transfection reagents may include, but are not limited to, cationic liposomes, lipids, polyamines, calcium phosphate, polyimide, polylysine, and/or combinations thereof. The transfection reagent may have a positive charge combined with a negative charge of nucleic acid. For example, cationic liposomes or polylysine complexes have net positive charges, allowing them to bind to DNA or RNA.

For example, the transfection reagent may contain a polymer, such as a DNA polycation complex. The transfection reagent may contain a cationic protein (e.g., histone, protamine) or a synthetic polymer (e.g., polylysine, polyarginine, DEAE glucan, polyphenylene ether, or polyethyleneimine). In certain embodiments, the transfection reagent includes a cationic lipid (e.g., a cationic liposome, such as Lipofectamine 2000 or Lipofectamine 3000).

Other substances that may be used as transfection reagents may include, for example, pH sensitive lipids, amphiphilic compounds, other liposomes, etc. In certain embodiments, the transfection reagent may include 1,2-dioleoyl-3-trimethylammonium-propane chloride (DOTMA), dioleoyl phosphatidylethanolamine (DOPE), dimethyl-2,3-dioleoxypropyl-2-(2-arginamido)ethylammonium trifluoroacetate (DOSPA), N-(2-arginine formyl)-N',N'-dioctadecyl glycamide (DOGS) and/or other substances that may form liposomes.

The kit may also include one or more cell culture mediums. For example, it may include a MEM medium (a minimum essential medium, a minimum basic medium or a low limit Eagle medium), etc. The content of the medium may be, for example, at least about 20µl, at least about 30µl, at least about 40µl, at least about 50µl, at least about 60µl, at least about 70µl, at least about 80µl, at least about 90µl, at least about 100µl, at least about 110µl, at least about 120µl, at least about 130µl, at least about 140µl, at least about 150µl, at least about 160µl, at least about 170µl, at least about 180µl, at least about 190µl, at least about 200µl, at least about 300µl, at least about 400µl, at least about 500µl, at least about 600µl or more.

### Cell treatment methods:

In a further aspect, the present application provides a method for delivering a nucleic acid to cells. The method may include: contacting the cells with the single-stranded DNA (e.g., the circular single-stranded DNA or the linear single-stranded DNA of the present application) containing the nucleic acid (e.g., a nucleic acid to be delivered) and the transfection reagent of the present application. A mass ratio of the single-stranded DNA to the transfection reagent may be about 5 to about 0.1. For example, the mass ratio of the single-stranded DNA to the transfection reagent may be about 4.5 to about 0.5, from about 4 to about 1, about 3.5 to about 1.5, about 3 to about 0.5, about 2.5 to about 0.1, about 2 to about 0.1, about 1.5 to about 0.1, about 1 to about 0.1, about 1 to about 0.2, about 1.5 to about 0.25, about 1.5 to about 0.3, about 1 to about 0.25, about 1.25 to about 0.2, about 0.75 to about 0.25, about 0.5 to about 0.3, etc.

In certain embodiments of the method, the mass ratio of the single-stranded DNA to the transfection reagent is about 5:1 to about 1:10. For example, the mass ratio of the single-stranded DNA to the transfection reagent may be about 4:1 to about 1:10, about 3:1 to about 1:10, about 2:1 to about 1:10, about 1:1 to about 1:10, about 1:1 to about 1:9, about 1:1 to about 1:8, about 1:1 to about 1:7, about 1:1 to about 1:6, about 1:1 to about 1:5, about 1:1 to about 1:4, about 1:1 to about 1:3, about 1:1 to about 1:2, about 1:1 to about 1:2, about 2:1 to about 1:2, about 2:1 to about 1:3, about 2:1 to about 1:4, about 2:1 to about 1:5, etc.

In a further aspect, the present application provides a method for expressing exogenous RNA and/or exogenous protein in cells. The method may include: introducing single-stranded DNA coding the exogenous RNA and/or the exogenous protein into the cells. The single-stranded DNA (e.g., circular single-stranded DNA or linear single-stranded DNA) may be the single-stranded DNA in the present application.

In certain embodiments of the method, the method also includes: culturing the cells under a condition that allows the expression of the exogenous RNA and/or the exogenous protein.

In a further aspect, the present application provides a method for reducing the cytotoxicity related to DNA transfection. The method may include: introducing the single-stranded DNA in the present application rather than the corresponding double-stranded DNA into the cells during transfection.

In a further aspect, the present application provides a method for reducing the cell immunogenicity related to DNA transfection. The method may include: introducing the single-stranded DNA in the present application rather than the corresponding double-stranded DNA into the cells during transfection.

In a further aspect, the present application provides a method for improving the efficiency of DNA transfection of cells. The method may include: introducing the single-stranded DNA in the present application rather than the corresponding double-stranded DNA into the cells during transfection.

In the method of the present application, "introducing the single-stranded DNA rather than the corresponding double-stranded DNA" usually means that it is basically not to introduce (e.g., in an amount that is difficult to accurately detect by ordinary methods) double-stranded DNA (e.g., plasmid DNA) with the same or corresponding (e.g., complementary or at least 80% homologous, at least 85% homologous, at least 90% homologous) nucleotide sequences into the cells. Instead, only by introducing the single-stranded DNA in the present application into the cells, the target RNA or target protein may be expressed in the cells.

In certain embodiments of the method in the present application, the step of introducing the single-stranded DNA into the cells includes: contacting the cells with the composition of the present application.

In certain embodiments of the method in the present application, the step of introducing the single-stranded DNA (e.g., circular single-stranded DNA or linear single-stranded DNA) into the cells includes: contacting the cells with the single-stranded DNA and the transfection reagent, wherein a mass ratio of the single-stranded DNA to the transfection reagent is about 5 to about 0.1. For example, the mass ratio of the single-stranded DNA to the transfection reagent may be about 4.5 to about 0.5, about 4 to about 1, about 3.5 to about 1.5, about 3 to about 0.5, about 2.5 to about 0.1, about 2 to about 0.1, about 1.5 to about 0.1, about 1 to about 0.1, about 1 to about 0.2, about 1.5 to about 0.25, about 1.5 to about 0.3, about 1 to about 0.25, about 1.25 to about 0.2, about 0.75 to about 0.25, about 0.5 to about 0.3, etc.

In certain embodiments of the method in the present application, the step of introducing the single-stranded DNA (e.g., circular single-stranded DNA or linear single-stranded DNA) into the cells includes: contacting the cells with the single-stranded DNA and the transfection reagent, wherein the mass ratio of the single-stranded DNA to the transfection reagent is about 5:1 to about 1:10. For example, the mass ratio of the single-stranded DNA to the transfection reagent may be about 4:1 to about 1:10, about 3:1 to about 1:10, about 2:1 to about 1:10, about 1:1 to about 1:10, about 1:1 to about 1:9, about 1:1 to about 1:8, about 1:1 to about 1:7, about 1:1 to about 1:6, about 1:1 to about 1:5, about 1:1 to about 1:4, about 1:1 to about 1:3, about 1:1 to about 1:2, about 1:1 to about 1:2, about 2:1 to about 1:2, about 2:1 to about 1:3, about 2:1 to about 1:4, about 2:1 to about 1:5, etc.

In certain embodiments of the method in the present application, the contact (e.g., contacting the cells with the single-stranded DNA and the transfection reagent, or contacting the cells with the composition in the present application) includes: incubating at about 20°C to about 45°C. For example, the incubation is carried out at about 22°C to about 44°C, at about 25°C to about 40°C, at about 27°C to about 38°C, at about 28°C to about 37°C, at about 30°C to about 36°C, at about 29°C to about 35°C , at about 31 °C to about 33°C, or at about 32°C to about 34°C.

In certain embodiments of the method in the present application, the contact includes: performing the incubation for at least 4 hours, for example, performing the incubation for at least 6 hours, at least 8 hours, at least 10 hours, at least 12 hours, at least 14 hours, at least 15 hours, at least 16 hours, at least 17 hours, at least 18 hours, at least 19 hours, at least 20 hours, at least 21 hours, at least 22 hours, at least 23 hours, at least 24 hours, at least 25 hours, at least 26 hours, at least 27 hours, at least 28 hours, at least 29 hours, at least 30 hours, at least 32 hours, at least 34 hours, at least 36 hours, at least 38 hours, at least 40 hours, at least 42 hours, at least 44 hours, at least 45 hours, at least 46 hours, at least 47 hours, at least 48 hours, at least 49 hours, at least 50 hours, at least 51 hours, at least 52 hours, at least 53 hours, at least 54 hours or more, etc.

The single-stranded DNA may include a nucleic acid sequence coding one or more RNAs and/or proteins. For example, the single-stranded DNA may be transcribed/reversely transcribed into RNA molecules (e.g., miRNA, mRNA or other RNA molecules) in cells as templates. In certain cases, the single-stranded DNA may be transformed into double-stranded DNA, which may exist in the cells freely, and may also be integrated into genomes of the cells (e.g., in a random insertion mode, or integrated into a specific site). The single-stranded DNA may be transcribed and translated to express the target protein (e.g., a therapeutic protein, a marker protein such as a fluorescent protein or a luminescent protein).

For example, the single-stranded DNA may include a nucleic acid sequence coding two or more RNAs and/or proteins. For example, the single-stranded DNA may include a first coding region and a second coding region, wherein the first coding region may encode a first RNA or a first protein, and the second coding region may independently encode a second RNA or a second protein that is the same as or different from the first RNA or the first protein. One or more non-coding regions may be included between the first coding region and the second coding region. In some cases, there is basically no non-coding region between the first coding region and the second coding region. In certain cases, the single-stranded DNA may contain a third coding region or more coding regions. The expression products of each coding region can form protein complexes or protein/nucleic acid complexes.

For example, the single-stranded DNA (for example, circular single-stranded DNA or linear single-stranded DNA) may include a nucleic acid sequence coding one or more RNAs and/or proteins (e.g., a nucleic acid sequence coding two or more RNAs and/or proteins), which may contain at least about 40 nucleotides, for example, at least about 50 nucleotides, at least about 100 nucleotides, at least about 200 nucleotides, at least about 300 nucleotides, at least about 400 nucleotides, at least 450 nucleotides, at least 500 nucleotides, at least 550 nucleotides, at least 600 nucleotides, at least 650 nucleotides, at least 700 nucleotides, at least 750 nucleotides, at least 800 nucleotides, at least 850 nucleotides, at least 900 nucleotides, at least 950 nucleotides, at least 1000 nucleotides, at least 1050 nucleotides, at least 1100 nucleotides, at least 1150 nucleotides, at least 1200 nucleotides, at least 1250 nucleotides, at least 1300 nucleotides, at least 1350 nucleotides, at least 1400 nucleotides, at least 1450 nucleotides, at least 1500 nucleotides, at least 1550 nucleotides, at least 1600 nucleotides, at least 1650 nucleotides, at least 1700 nucleotides, at least 1750 nucleotides, at least 1800 nucleotides, at least 1850 nucleotides, at least 1900 nucleotides, at least 1950 nucleotides, at least 2000 nucleotides, at least 2100 nucleotides, at least 2200 nucleotides, at least 2300 nucleotides, at least 2400 nucleotides, at least 2500 nucleotides, at least 2600 nucleotides, at least 2700 nucleotides, at least 2800 nucleotides, at least 2900 nucleotides, at least 3000 nucleotides, at least 3100 nucleotides, at least 3200 nucleotides, at least 3300 nucleotides, at least 3400 nucleotides, at least 3500 nucleotides, at least 3600 nucleotides, at least 3700 nucleotides, at least 3800 nucleotides, at least 3900 nucleotides, at least 4000 nucleotides, at least 4100 nucleotides, at least 4200 nucleotides, at least 4300 nucleotides, at least 4400 nucleotides, at least 4500 nucleotides, at least 4600 nucleotides, at least 4700 nucleotides, at least 4800 nucleotides, at least 4900 nucleotides, at least 5000 nucleotides, at least 5100 nucleotides, at least 5200 nucleotides, at least 5300 nucleotides, at least 5400 nucleotides, at least 5500 nucleotides, at least 5600 nucleotides, at least 5700 nucleotides, at least 5800 nucleotides, at least about 5900 nucleotides, at least about 6000 nucleotides or more.

For example, the single-stranded DNA may contain a sense strand (also known as a coding strand, a sense strand, a positive strand, a nontemplete strand or a +strand) nucleic acid sequence coding RNA and/or protein. For example, the nucleic acid sequence contained in the single-stranded DNA is basically consistent with a coding sequence of a target protein expressed (e.g., a sequence of mRNA coding the target protein).

In certain embodiments, the single-stranded DNA includes an antisense strand nucleic acid sequence coding RNAs and/or proteins. For example, the antisense strand nucleic acid sequence and the sense strand sequence (e.g., the coding nucleic acid sequence of the target protein) are basically complementary. In certain embodiments, the antisense strand nucleic acid sequence and the sense strand sequence are completely complementary. For example, a nucleotide sequence of an EGFP cssDNA antisense strand sequence (2Ambystoma laterale × Ambystoma jeffersonianum) of the present application may be shown as SEQ ID NO:7.

The single-stranded DNA of the present application may also include other elements, such as a promoter sequence (e.g., a CMV promoter), an enhancer sequence and/or a polyA sequence (e.g., SV40 polyA).

The "transfection reagent" may refer to a substance (e.g., a compound) used to mediate a nucleic acid into cells. For example, the transfection reagents may include, but are not limited to, cationic liposomes, lipids, polyamines, calcium phosphate, polyimide, polylysine, and/or combinations thereof. The transfection reagent may have positive charges combined with negative charges of the nucleic acid. For example, cationic liposomes or polylysine complexes have net positive charges, allowing them to bind to DNA or RNA.

For example, the transfection reagent may include a polymer, such as a DNA polycation complex. The transfection reagent may contain a cationic protein (e.g., histone, protamine) or a synthetic polymer (e.g., polylysine, polyarginine, DEAE glucan, polyphenylene ether, polyethyleneimine, etc.). In contain embodiments, the transfection reagent includes a cationic lipid (e.g., a cationic liposome, such as Lipofectamine 2000 or Lipofectamine 3000).

Other substances that may be used as the transfection reagents may include, for example, pH sensitive lipids, amphiphilic compounds, other liposomes, etc. In certain embodiments, the transfection reagent may include 1,2-dioleoyl-3-trimethylammonium-propane chloride (DOTMA), dioleoyl phosphatidylethanolamine (DOPE), dimethyl-2,3-dioleoxypropyl-2-(2-arginamido)ethylammonium trifluoroacetate (DOSPA), N-(2-arginine formyl)-N',N'-dioctadecyl glycamide (DOGS) and/or other substances that may form liposomes.

The method may also include: applying one or more cell culture mediums. For example, the one or more cell culture mediums may include a MEM medium (a minimum essential medium, a minimum basic medium or a low limit Eagle medium), etc. The content of the medium may be, for example, at least about 20µl, at least about 30µl, at least about 40µl, at least about 50µl, at least about 60µl, at least about 70µl, at least about 80µl, at least about 90µl, at least about 100µl, at least about 110µl, at least about 120µl, at least about 130µl, at least about 140µl, at least about 150µl, at least about 160µl, at least about 170µl, at least about 180µl, at least about 190µl, at least about 200µl, at least about 300µl, at least about 400µl, at least about 500µl, at least about 600µl or more. Before application, the culture medium may be incubated with the single-stranded DNA of the present application and/or the transfection reagent of the present application (e.g., incubation is performed for at least about 5min, at least about 10min, at least about 12min, at least about 13min, at least about 14min, at least about 15min, at least about 20min, at least about 25min, at least about 30min, at least about 35min or longer). For example, the incubation is performed at about 20°C to about 45°C. For example, the incubation is performed at about 22°C to about 44°C, at about 25°C to about 40°C, at about 27°C to about 38°C, at about 28°C to about 37°C, at about 30°C to about 36°C, at about 29°C to about 35°C, at about 31 °C to about 33°C , or at about 32°C to about 34°C.

In certain embodiments, the content of the single-stranded DNA (e.g., circular single-stranded DNA, or linear single-stranded DNA) used is about 10ng to about 1500ng. For example, this content may be about 10ng to about 1400ng, about 10ng to about 1300ng, about 10ng to about 1200ng, about 10ng to about 1100ng, about 10ng to about 1000ng, about 10ng to about 950ng, about 10ng to about 900ng, about 10ng to about 850ng, about 10ng to about 800ng, about 10ng to about 750ng, about 10ng to about 700ng, about 10ng to about 650ng, about 10ng to about 600ng, about 10ng to about 550ng, about 10ng to about 500ng, about 10ng to about 450ng, about 10ng to about 400ng, about 10ng to about 350ng, about 10ng to about 300ng, about 10ng to about 250ng, about 10ng to about 200ng, about 10ng to about 150ng, about 10ng to about 100ng, about 10ng to about 50ng, etc.

In certain embodiments, the concentration of the single-stranded DNA (e.g., circular single-stranded DNA, or linear single-stranded DNA) used is about 0.1nM to about 5nM. For example, this concentration may be about 0.2nM to about 4.5nM, about 0.3nM to about 4nM, about 0.4nM to about 3.5nM, about 0.5nM to about 3nM, about 0.6nM to about 2.5nM, about 0.7nM to about 2nM, about 0.8nM to about 1.9nM, about 0.9nM to about 1.8nM, about 1nM to about 1.6nM, about 1.1nM to about 1.5nM, about 1.2nM to about 1.4nM, about 1.3nM to about 1.7nM, etc.

In certain embodiments, the amount of the transfection reagent used is about 10ng to about 4500ng. For example, this amount is about 10ng to about 4000ng, about 10ng to about 3800ng, about 10ng to about 3600ng, about 10ng to about 3500ng, about 10ng to about 3300ng, about 10ng to about 3000ng, about 10ng to about 2800ng, about 10ng to about 2600ng, about 10ng to about 2400ng, about 10ng to about 2200ng, about 10ng to about 2000ng, about 10ng to about 1900ng, about 10ng to about 1800ng, about 10ng to about 1700ng, about 10ng to about 1600ng, about 10ng to about 1500ng, about 10ng to about 1400ng, about 10ng to about 1300ng, about 10ng to about 1200ng, about 10ng to about 1100ng, about 10ng to about 1000ng, about 10ng to about 950ng, about 10ng to about 900ng, about 10ng to about 850ng, about 10ng to about 800ng, about 10ng to about 750ng, about 10ng to about 700ng, about 10ng to about 650ng, about 10ng to about 600ng, about 10ng to about 550ng, about 10ng to about 500ng, about 10ng to about 450ng, about 10ng to about 400ng, about 10ng to about 350ng, about 10ng to about 300ng, about 10ng to about 250ng, about 10ng to about 200ng, about 10ng to about 150ng, about 10ng to about 100ng, about 10ng to about 50ng, etc.

In certain embodiments, the method described in the present application is an *in vivo* method or an *in vitro* method.

In certain embodiments of the method described in the present application, the cells are somatic cells, tumor cells and/or immune cells.

For example, the cells may be selected from: lung cancer cells, kidney cells, melanoma cells, liver cancer cells, cervical cancer cells, glioma cells, epithelial cells, colon cancer cells, fibroblasts, breast cancer cells, stomach cancer cells and immune effector cells.

In certain cases, the cells may be selected from: A549 cells, C6 cells, MCF-7 cells, HepG2 cells, CT26 cells, Hela cells, Helf cells, MDCK cells, B16 cells, Hep3B cells, L929 cells, 4T1 cells, 293T cells, MGC803 cells and macrophages.

### Cell, organism or part thereof:

In a further aspect, the present application provides cells obtained by processing with the method according to any aspect of the present application.

For example, the single stranded DNA rather than the corresponding double-stranded DNA of the present application is introduced into the cells. For example, the cells are brought into contact with the composition of the present application. For example, the single-stranded DNA described in the present application is introduced into the cells to express exogenous RNA and/or exogenous protein.

In a further aspect, the present application provides an organism (e.g., non-human organism) or components thereof, which may include the cells described in the present application (e.g., cells treated by the method of the present application).

In certain embodiments, the components are selected from tissues (e.g., tumor tissues, etc.), organs, and combinations thereof.

### Use:

In a further aspect, the present application provides use of single-stranded DNA in the preparation of a composition (e.g., a transfection composition, such as a cell transfection composition, or nucleic acid delivery composition). The composition may be used for one or more of the followings: 1) delivering at least part of nucleic acid contained in the single-stranded DNA of the present application to cells; 2) expressing exogenous RNA or exogenous protein encoded by the single-stranded DNA in the cells; 3) improving the DNA transfection efficiency of the cells; 4) reducing the cytotoxicity related to DNA transfection; and/or 5) reducing the cellular immunogenicity related to DNA transfection. The composition may be the composition (e.g., the transfection composition) described in any aspect of the present application.

In certain cases, the single-stranded DNA or the composition of the present application may be used to prepare drugs (e.g., nucleic acid drugs), such as gene therapy and/or nucleic acid vaccine.

In certain embodiments, the cells are somatic cells, tumor cells, and/or immune cells.

In certain embodiments, the cells are selected from: lung cancer cells, kidney cells, melanoma cells, liver cancer cells, cervical cancer cells, glioma cells, epithelial cells, colon cancer cells, fibroblasts, breast cancer cells, stomach cancer cells, and immune effector cells.

In certain embodiments, the cells are selected from: A549 cells, C6 cells, MCF-7 cells, HepG2 cells, CT26 cells, Hela cells, Helf cells, MDCK cells, B16 cells, Hep3B cells, L929 cells, 4T1 cells, 293T cells, MGC803 cells, and macrophages.

The present application also relates to the following contents.

The invention of the present application provides an application of single-stranded DNA in gene transfection, specifically including the application of circular single-stranded DNA generated based on traditional phage vectors and linear single-stranded DNA obtained by an asymmetric PCR method in gene transfection. Different from the traditional circular double-stranded plasmid transfection, the transfection efficiency of the circular single-stranded DNA is proportional to the concentration of the transfection reagent. At the same concentration, the circular single-stranded DNA is more easily delivered by the transfection reagent to the cells that are difficult to transfect, and the expression amount is higher, resulting in lower cytotoxicity. Circular single-stranded DNA can significantly reduce the cytotoxicity caused by polymers, which effectively improves the transfection efficiency, and does not significantly cause the cell toxicity. In addition, linear single-stranded DNA also proves the feasibility of gene transfection. Therefore, by means of gene transfection using single-stranded DNA, the expression of related genes can be seen. Thus, single-stranded DNA can be used as a vector for gene transfection.

In certain aspects, the present application provides an application of single-stranded DNA in gene transfection.

The single-stranded DNA includes circular single-stranded DNA generated based on bacteriophage vectors and linear single-stranded DNA generated by asymmetric PCR. The single-stranded DNA and the transfection reagent may be incubated and then cultured for 24-48 hours in a cell culture dish.

For example, the nucleic acid number of the single-stranded DNA may be 1000-50000nt.

For example, the transfection reagent may be selected from a cationic polymer. In certain embodiments, the transfection reagent is selected from more than one of Lipofectamine 2000 or polyetherimide.

A preparation method of the circular single-stranded DNA may include the following steps: 1) inserting a gene linker into a vector to obtain a gene vector; 2) co-transforming the gene vector and an auxiliary phage vector into competent cells; and 3) culturing the circular single-stranded DNA with a single band by culturing for different times according to different lengths of single-stranded DNA. In step 2), wherein the phage vector may be an M13-based plasmid vector.

A preparation method of the linear single-stranded DNA may include the following steps: (a) designing upstream primers and downstream primers based on genes; (b) performing a PCR reaction by controlling a proportion of the upstream primers to the downstream primers; and (c) separating and purifying a PCR product by agarose gel electrophoresis. In step (a), the upstream primer sequence may be as shown in SEQ ID NO: 3, and the downstream primer sequence may be as shown in SEQ ID NO: 4. In certain cases, in step (a), a molar ratio of the upstream primers to the upstream primers may be 50:1.

For example, in view of the shortcomings of the prior art, the invention of the present application provides an application of single-stranded DNA (ssDNA) in gene transfection. Specifically, in circular single-stranded DNA based on a pScaf vector, a target gene is inserted into the pScaf vector to obtain circular single-stranded DNA (cssDNA) with a single band by controlling the culture time. Linear single-stranded DNA (lssDNA) is produced by a traditional asymmetric PCR method. The resulting lssDNA and cssDNA each contain CMV enhancer and promoter, a target gene, SV40 polyA and other sequences, thereby reducing the introduction of other unrelated sequences, so that the length of DNA fragments can be shortened to the maximum extent.

In one aspect, the present application provides an application of single-stranded DNA in gene transfection.

For example, the single-stranded DNA may include circular single-stranded DNA generated based on phage vectors and linear single-stranded DNA generated by asymmetric PCR. After incubation of the single-stranded DNA and a transfection reagent, the single-stranded DNA may be put into a cell culture dish for 24-48 hours, and the obvious expression of the target protein can be seen.

For another example, the nucleic acid number of the single-stranded DNA may be synthesized to a length of 1000-50000nt. For example, the transfection reagent may be selected from a cationic polymer, such as Lipofectamine 2000 (lip 2000) or polyetherimide (PEI).

For example, a preparation method of cssDNA generated based on a pScaf vector may include the following steps: (1) inserting a target gene into the pScaf vector through an enzyme digestion site linker to obtain a pScaf-target gene vector; (2) co-transforming 10-20ng of pScaf-target gene vector and 20-40ng of M13-based auxiliary phage vector (auxiliary plasmid pSB4423) to 100µL of XL1 Blue competent cells, with a culture temperature of 30°C; (3) picking up monoclone and activating it in 1mL medium for 1h, then transferring to 5mL liquid medium, and performing expanding culture at 30°C for 4-5 hours; (4) putting the bacterial solution obtained in step (3) in 200mL of 2×YT culture medium and culturing for 8-24 h, and controlling the culture time according to the length of the target gene to obtain a target product with a single band and no hetero band; (5) measuring a concentration of the target product using nanodrop, and calculating a molar concentration of the target product according to the molecular weight; and (6) performing gene transfection using the cssDNA obtained in step (5).

In step (1), the target gene may include an EGFP gene and an EGFP-mCherry gene. The EGFP gene sequence may be as shown in SEQ ID NO: 1. The EGFP-mCherry gene sequence may be shown as SEQ ID NO: 2.

In step (1) of the method, the digestion sites may be Kpnl (GGTACC) and BamHI (GGATCC). In step (2) of the method, a pScaf target gene vector may be used to integrate an M13 replicon with a start site and a stop site of single-stranded DNA, thereby reducing the generation of by-products.

In step (4) of the method, the DNA obtained by controlling the culture time may be determined as circular single-stranded DNA through S1 nuclease verification.

For example, the preparation method of lssDNA based on traditional asymmetric PCR may include the following steps: (a) taking a pEGFP-N1 plasmid as a template, and performing a PCR reaction using PrimerSTAR (R045A, TAKARA) enzyme of Takara Company as well as designed upstream primer and downstream primer; (b) performing agarose gel electrophoresis on the resulting PCR product; (c) cutting a target strip off according to a DNA marker position; (d) recovering the target DNA; (e) detecting the concentration of lssDNA using nanodrop; and (f) performing gene transfection using the obtained lssDNA. For example, in step (a), the EGFP gene sequence may be as shown in SEQ ID NO: 1. For example, in step (a), the upstream primer sequence may be as shown in SEQ ID NO: 3, and the downstream primer sequence may be as shown in SEQ ID NO: 4. For example, a molar ratio of the upstream primer to the upstream primer may be 50:1.

The beneficial effects of the invention of the present application may include: the invention of the present application applies ssDNA in gene transfection, which is different from the traditional circular double-stranded plasmid transfection; the transfection efficiency of cssDNA is proportional to the concentration of DNA and the concentration of the transfection reagent, such that cssDNA of the same concentration is easier to be delivered by the transfection reagent to cells that are more difficult to transfect, and the expression amount is higher, resulting in lower cytotoxicity; cssDNA can significantly reduce the cytotoxicity caused by polymers while commercial cationic polymer transfection reagents are used for transfection, which effectively improves the transfection efficiency, and does not significantly cause the cell toxicity; and in addition, lssDNA has also proved the feasibility of being used for gene transfection. Therefore, it is proved that ssDNA can be used as a potential vector for gene transfection and gene delivery.

Without being limited by any theory, the examples set forth below are only intended to illustrate the methods and systems of the present application, and are not intended to limit the scope of the invention of the present application.

### EXAMPLES

### Example 1. Obtaining circular single-stranded DNA

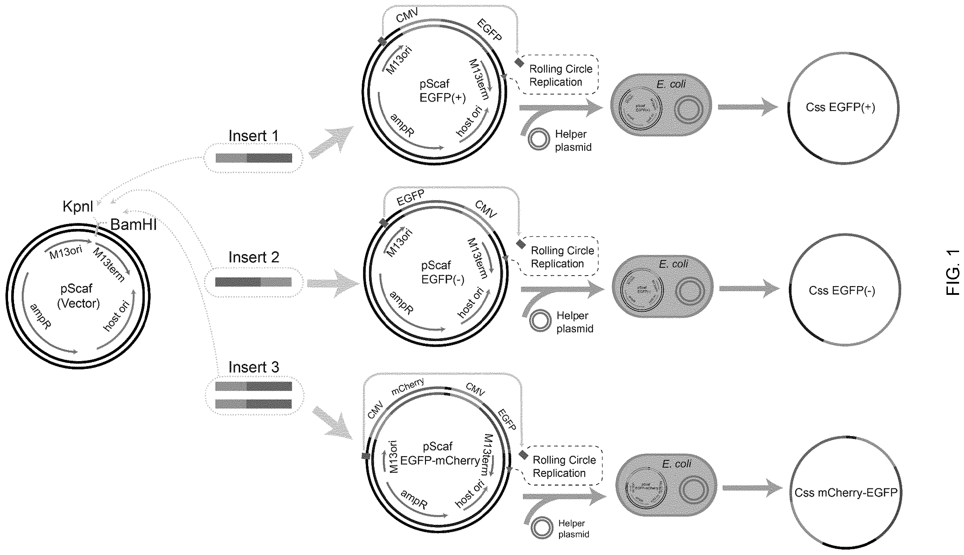

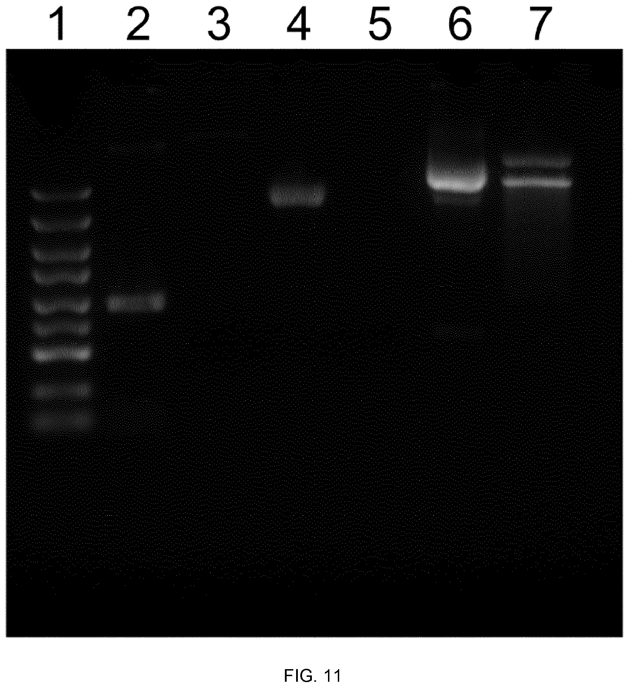

An expression vector is constructed and the circular single-stranded DNA is obtained by the following steps:
1) performing restriction digestion on a pScaf vector (Addgene plasmid # 111401) by Kpnl and BamHI restriction endonucleases, and then linking an EGFP gene fragment and an EGFP-mCherry gene fragment amplified by PCR respectively to the pScaf vector to obtain a pScaf-EGFP vector and a pScaf-mCherry-EGFP vector (as shown in FIG.1);
2) co-transforming10ng of pScaf-EGFP vector, 10ng of pScaf-EGFP-mCherry vector and 15ng of M13-based pSB4423 plasmid (donated to Ke yonggang laboratory in the United States) into XL1-Blue competent cells, coating a plate and culturing at 30 °C for 16h;
3) picking up monoclone and putting into 1mL of 2×YT medium (containing 5mM MgCl₂, 100µg/mL ampicillin sodium, 20µg/mL chloramphenicol), and culturing at 30°C and 220 rpm for 1h;
4) inoculating the culture in step 3) to 5mL ; 2×YT medium (containing 5mM MgCl₂, 100µg/mL ampicillin sodium, 20µg/mL chloramphenicol) and culturing at 30°C and 220 rpm for 4h;
5) inoculating the culture in step 4) to 200mL 2×YT medium (containing 5mM MgCl₂, 100µg/mL ampicillin sodium, 20µg/mL chloramphenicol), and continuing to culture at 30 °C and 220 rpm, in which pScaf-EGFP is cultured for 8h and pScaf-EGFP-mCherry is cultured for 12h;
6) centrifuging the culture in step 5) at 4°C and 8000×rcf for 10min, and collecting the supernatant;
7) adding 3.2g of PEG-8000 and 2.4g of NaCl into the supernatant obtained in step 6), mixing them well, and placing them in an ice bath for 30min;
8) centrifuging the mixture of step 7) at 10000×rcf and 4°C for 30min to obtain a white precipitate;
9) performing resuspension on the precipitate obtained in step 8) using 1.5mL of 10mM Tris (pH=8.5), and centrifuging at 4°C and 10,000×rcf for 15min;
10) repeating step 9) three times to remove residual bacteria;
11) adding 3mL of cracking solution (0.2mol/L NaOH, 1% SDS) to the solution obtained in step 10), mixing it upside down, and cracking for 5min at room temperature;
12) adding 2.5mL of termination solution (3mol/L potassium acetate, pH=5.5) to the solution obtained in step 11), mixing it upside down, and putting in an ice water bath for 15min;
13) centrifugating the solution obtained in step 12) at 10000×rcf and 4°C for 30min to remove the precipitate, and collecting the supernatant;
14) adding two times of volumes of absolute ethanol into the supernatant obtained in step 13), and placing the mixed solution at -20 °C for precipitation overnight;
15) centrifugating the solution obtained in step 14) at 10000×rcf and 4°C for 30min to obtain EGFP circular single-stranded DNA (EGFP cssDNA) (its nucleic acid sequence is shown in SEQ ID NO: 5) and EGFP-mCherry circular single-stranded DNA (EGFP-mCherry cssDNA) (its nucleic acid sequence is shown in SEQ ID NO: 6);
16) washing the EGFP cssDNA and EGFP-mCherry cssDNA with 3mL of 75% ethanol;
17) quantitatively determining the EGFP cssDNA and EGFP-mCherry cssDNA using nanodrop;
18) adding single-stranded DNA enzyme (S1 nuclease) to the obtained EGFP cssDNA and verifying by agarose gel electrophoresis (the results are shown in FIG.11). Briefly, 2µg of the EGFP cssDNA, 2µg of P7560 (Scaffold DNA, Company: IDT, Article No.: 1081309), and 2µg of EGFP plasmid double-stranded DNA (pEGFP-N1-4x, Addgene plasmid #172283) are taken respectively and incubated with S1 nuclease at 37°C for 30min. S1 nuclease is extracted from *aspergill suoryzae* and can specifically degrade single-stranded DNA. As shown in FIG.11, the EGFP cssDNA and P7560 positive control (phage circular single-stranded DNA) may be rapidly degraded by S1 nuclease, while the double-stranded plasmid DNA cannot be degraded. Therefore, it can be determined that the EGFP cssDNA prepared in this example is single-stranded DNA. Similarly, it is confirmed that the EGFP-mCherry cssDNA is also single-stranded DNA. As shown in FIG.11, a first lane shows a 5kb DNA marker, a second lane is EFGP cssDNA treated without S1 nuclease, a third lane is EFGP cssDNA treated with S1 nuclease, a fourth lane is P7560 treated without S1 nuclease, a fifth lane is P7560 treated with S1 nuclease, a sixth lane is EGFP double-stranded plasmid DNA treated without S1 nuclease, and a seventh lane is EGFP double-stranded plasmid DNA treated with S1 nuclease.

### Example 2. Delivering the single-stranded DNA of Example 1 to cells by electroporation and detecting its expression

The single-stranded DNA is delivered to the cell by the following steps:
1) setting the required pulse conditions on an electroporation system Gene PµLser Xcell;
2) adding 20µg of EGFP cssDNA obtained in Example 1 to an electroporation cup;
3) adding cells to the electroporation cup, and tapping one side of the electroporation cup to mix; specifically, adding 200µL of 5×10⁶ cells to the 0.2cm electroporation cup;
4) putting the electroporation cup into an electroporation chamber, covering, and pulsing once;
5) adding 0.5ml of cell culture medium intermediately after the pulsing, and then transferring the cells to a culture dish with a Pap pipette;
6) shaking the dish gently to ensure that the cells are evenly distributed on the surface of the dish, and culturing the cells in an incubator at 37°C; and
7) within 24-48h after electroporation, detecting the expression of the single-stranded DNA.

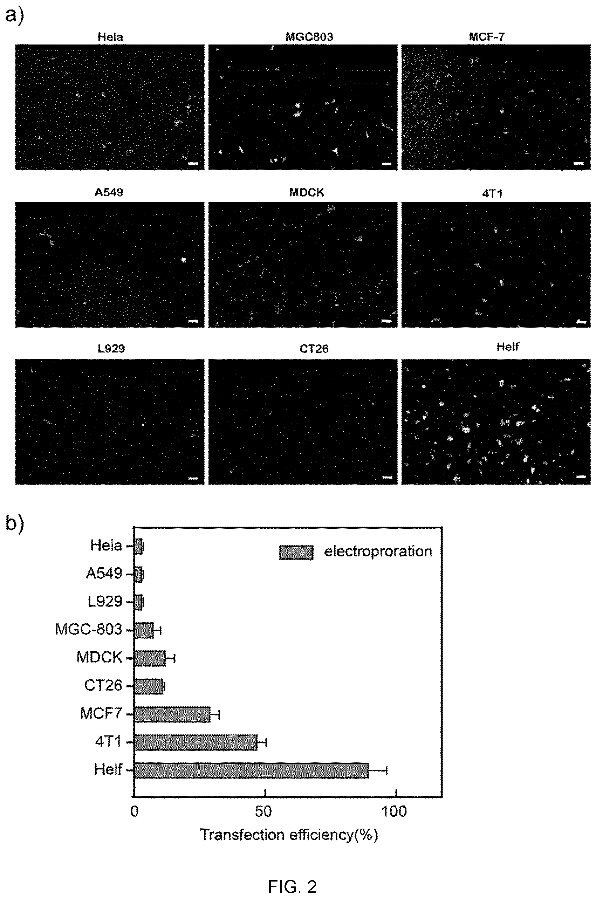

In this example, the expression of the EGFP cssDNA in a variety of cell lines (Hela cells, A549 cells, L929 cells, MGC803 cells, MDCK cells, CT26 cells, MCF7 cells, 4T1 cells and Helf cells) is verified. After 24h of culture, we observed that EGFP cssDNA can be expressed in these cell lines (the results are shown in FIGS. 2A-2B). FIG. 2A shows a fluorescent expression image of EGFP cssDNA in each cell line. FIG. 2B shows the electroporation transfection/expression efficiency of EGFP cssDNA in various cell lines. It can be seen that the circular single-stranded DNA of the present application can effectively express the target protein in a variety of cell lines.

### Example 3. Delivering the single-stranded DNA of Example 1 to cells with transfection reagents and detecting its expression

The single-stranded DNA is delivered to the cells by the following steps:
1) mixing 1,5pg of cationic polymer (Lipofectamine 2000, Thermophile, Article No. 11668500) with a MEM culture medium at room temperature and placing for 5min (the total volume is 50µL);
2) mixing 1.3nM (50µL, about 460ng) sense strand EGFP cssDNA prepared in Example 1 with 1,5pg of cationic polymer (Lipofectamine 2000) mixed well in 50µL MEM medium in step 1), and gently blowing with a pipette gun to obtain 100µL of well-mixed solution and placing at room temperature for 15min;
3) mixing 1.3nM (50µL, about 460ng) antisense strand EGFP cssDNA prepared in Example 1 with 1.5µg of cationic polymer (Lipofectamine 2000) mixed well in 50µL of MEM medium in step 1), and gently blowing with the pipette gun to obtain 100µL of well-mixed solution at room temperature for 15min;
4) mixing 1.3nM (50µL, about 460ng) EGFP-mCherry cssDNA prepared in Example 1 with 1,5pg of cationic polymer (Lipofectamine 2000) mixed well in 50µL of MEM medium in step 1), and gently blowing with the pipette gun to obtain 100µL of well-mixed solution at room temperature for 15min;
5) pre-inoculating cells (1×10⁵ cells) in a 24 well plate and culturing in an incubator for 12h;
6) adding dropwise the above mixed solutions to the cells laid on the 24 well plate in advance and culturing them for 24h; and
7) observing the transfection efficiency of cells using a fluorescence microscope.

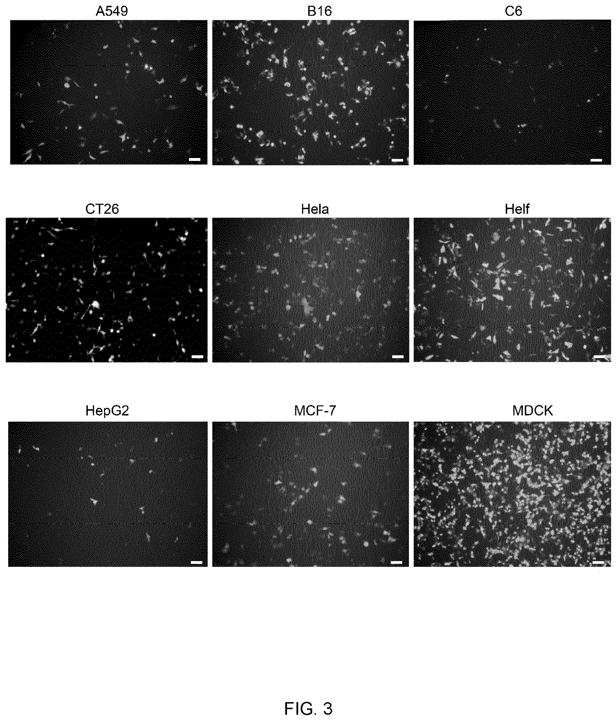

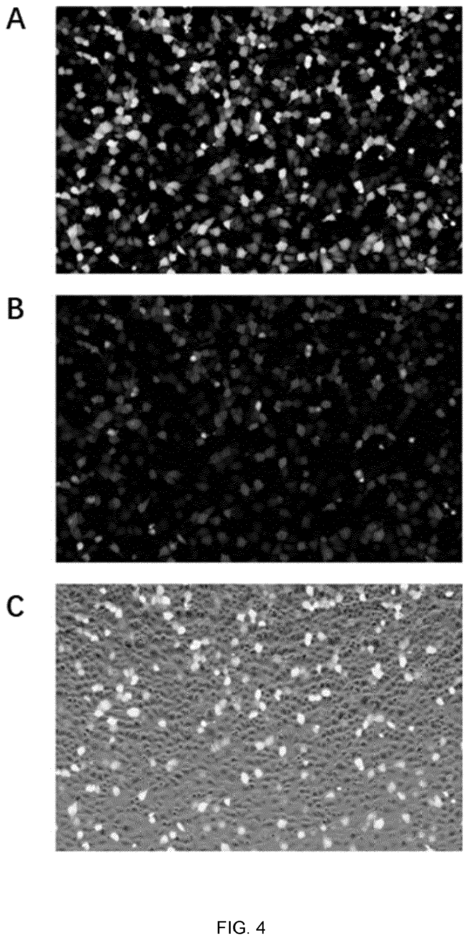

In this example, the transfection efficiency and expression of the EGFP cssDNA (sense strand or antisense strand) and EGFP-mCherry cssDNA in a variety of cell lines (such as A549 cells, C6 cells, MCF-7 cells, HepG2 cells, CT26 cells, Hela cells, Helf cells and MDCK cells) are verified after mixed with transfection reagents respectively. After 24h of culture, it is observed that the sense strand and antisense strand of EGFP cssDNA can be expressed in these cell lines (the results are shown in FIG.3), and long strand single-stranded DNA containing multiple protein coding regions (e.g., EGFP-mCherry cssDNA in this example) may also be effectively transfected and expressed (the results are shown in FIGS. 4A-4C). FIG. 4A shows the expression of EGFP protein in EGFP-mCherry cssDNA after transfection. FIG. 4B shows the expression of mCherry protein in EGFP-mCherry cssDNA after transfection. FIG. 4C shows the superposition of EGFP protein expression and mCherry protein expression, showing their coexpression.

### Example 4. Comparison of transfection efficiency between single-stranded DNA and double-stranded plasmid DNA

Nucleic acid transfection is carried out through the following steps (the difference of transfection efficiency is tested under different ratios of DNA: transfection reagents):
1) Ratio 1: adding 0.5µg of cationic polymer (Lipofectamine 2000) into 50µL of MEM medium, mixing well at room temperature and placing for 5min; subsequently, mixing 1.3nM EGFP cssDNA (in 50µL MEM medium, about 460ng) and 1.3nM corresponding double-stranded plasmid DNA (in 50µL MEM medium, about 460ng) respectively with 0.5µg of prepared cationic polymer (Lipofectamine 2000, already in 50µL MEM medium) to obtain 100µL of well-mixed transfection composition, and storing at room temperature for 15min;
2) Ratio 2: adding 1µg of cationic polymer (Lipofectamine 2000) into 50µL of MEM medium, mixing well at room temperature and placing for 5min; subsequently, mixing 1.3nM EGFP cssDNA (in 50µL MEM medium, about 460ng) and 1.3nM corresponding double-stranded plasmid DNA (in 50µL MEM medium, about 460ng) respectively with 1µg of prepared cationic polymer (Lipofectamine 2000, already in 50µL MEM medium) to obtain 100µL of well-mixed transfection composition, and storing at room temperature for 15min;
3) Ratio 3: adding 2µg of cationic polymer (Lipofectamine 2000) into 50µL of MEM medium, mixing well at room temperature and placing for 5min; subsequently, mixing 1.3nM EGFP cssDNA (in 50µL MEM medium, about 460ng) and 1.3nM corresponding double-stranded plasmid DNA (in 50µL MEM medium, about 460ng) respectively with 2µg of the prepared cationic polymer (Lipofectamine 2000, already in 50µL MEM medium) to obtain 100µL of well-mixed transfection composition, and storing at room temperature for 15min;
4) pre-inoculating cells (1×10⁵ cells) in a 24 well plate and culturing in an incubator for 12h;
5) adding dropwise the above mixed solutions to the cells laid on the 24 well plate in advance and culturing them for 24h; and
6) observing the transfection efficiency of cells using a fluorescence microscope.

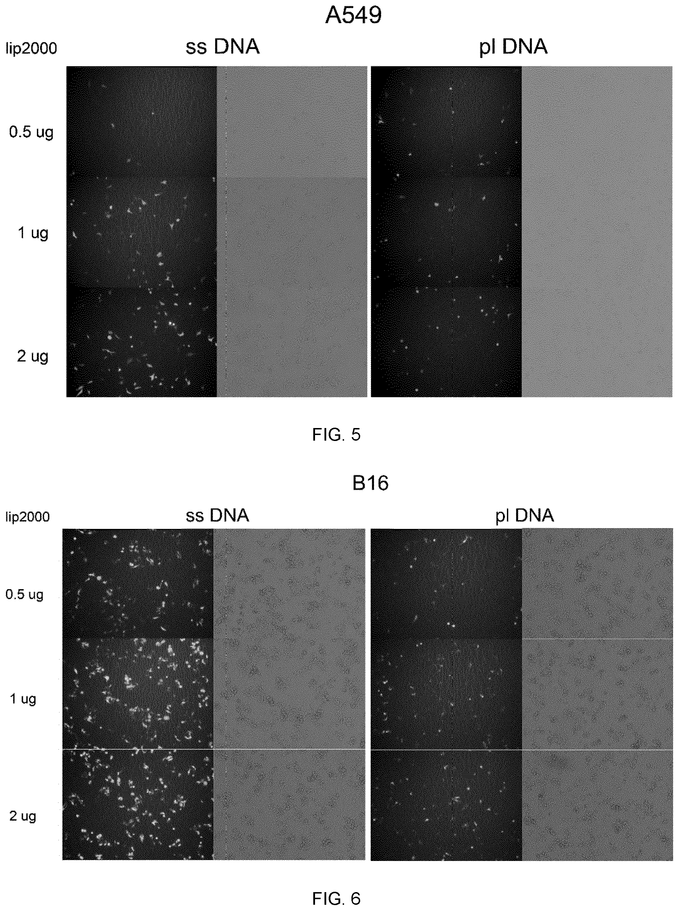

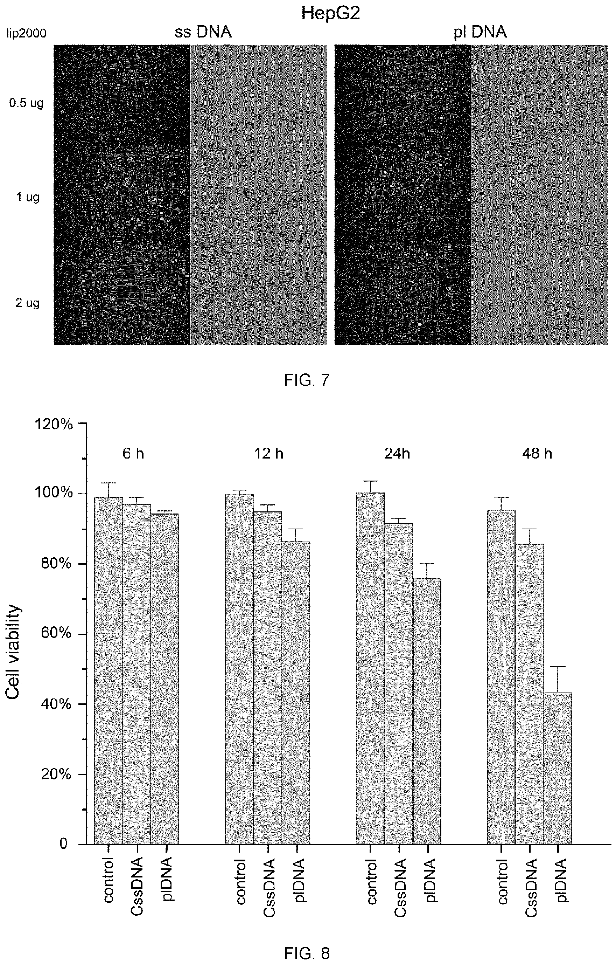

In this example, the transfection efficiency and expression of the EGFP cssDNA in a variety of cell lines (such as A549 cells, B16 cells and HepG2 cells, including cell lines that are difficult to transfect) are verified. When the same concentration of DNA is used, the transfection efficiency of single-stranded DNA is significantly higher than that of double-stranded plasmid DNA. In addition, the transfection efficiency of single-stranded DNA can be improved by using a higher proportion of transfection reagents (the results are shown in FIGS. 5-7). FIG. 5 shows a transfection effect in A549 cells. FIG. 6 shows a transfection effect in B16 cells, and FIG. 7 shows a transfection effect in HepG2 cells.

### Example 5. Comparison of cytotoxicity between single-stranded DNA and double-stranded plasmid DNA after transfection

The experimental steps are briefly described as follows:
1) preparing a cell suspension and counting the cells;
2) inoculating MDCK cells into a 96 well plate: coating the plate at the density of 5×10³ cells/well, adding about 100µL of cell suspension into each well, and repeating the same sample for 5 times;
3) culturing in an incubator at 37°C for 12-24h;
4) adding single-stranded DNA (EGFP cssDNA) and double-stranded plasmid DNA to form complexes with a transfection reagent Lipofectamine2000 (DNA: 600ng; Lipofectamine2000: 3µg) respectively;
5) culturing at 37°C in the incubator for 6h, 12h, 24h and 48h respectively;
6) adding 10µL of CCK8 reagent for toxicity analysis;
7) incubating in the incubator at 37°C for 4h; and
8) mixing gently on a vibrator for 1min to ensure uniform color distribution, measuring the absorbance of each well at 450nm using a microplate reader, and then performing data analysis and processing.

In this example, the cytotoxicity of the composition of the single-stranded DNA and a large number of transfection reagents during cell transfection is verified, and compared with that of the corresponding double-stranded plasmid DNA transfection composition.

As shown from the results of this example that under the same DNA concentration, under the action of a high-concentration transfection reagent (e.g., Lipofectamine 2000), after 0-48h of transfection, it can be seen that the plDNA group has obvious cytotoxicity compared with the single-stranded DNA group after 24h, while the single-stranded DNA group does not cause obvious cytotoxicity (the results are shown in FIG. 8). It can be seen that the corresponding double-stranded plasmid DNA can be replaced by single-stranded DNA to reduce the cytotoxicity related to DNA transfection. In addition, it is surprising that when single-stranded DNA is mixed with a large number of transfection reagent, a synergistic effect can be shown. In the one hand, the cytotoxicity of larger dose of transfection reagents is reduced, and in the other hand, the transfection efficiency of single-stranded DNA is improved.

### Example 6 Transfection effect of linear single-stranded DNA

The experimental steps are briefly described as follows:

### 6.1 Obtaining linear single-stranded DNA (EGFP lssDNA)

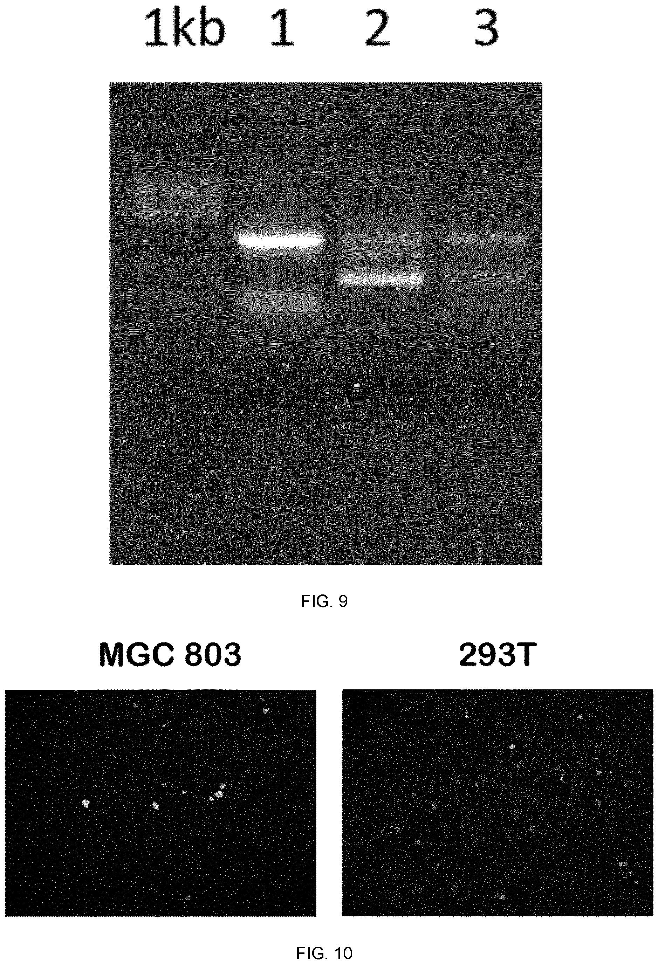

1) A PCR reaction system is prepared according to the following formula:
   Reverse product amplification: pEGFP-N1 plasmid (Addgene plasmid # 54767): 2µL (10ng/µL), forward primer: 2µL (0.2µM), reverse primer: 2µL (10µM), PrimeSTAR Max Premix (25µL, R045A, Takara), ddH₂O: 19µL.
   Forward product amplification: pEGFP-N1 plasmid: 2µL (10ng/µL), forward primer: 2µL (10µM), reverse primer: 2µL (0.2µM), PrimeSTAR Max Premix (25µL, R045A, Takara), ddH₂O: 19µL.
   The forward primer sequence in forward product amplification and reverse product amplification is shown in SEQ ID NO: 3, and the reverse primer sequence is shown in SEQ ID NO: 4.
2) Amplification is performed according to the following reaction procedure:
   reaction is performed at 95°C for 10min, at 95°C for 30s, at 60°C for 30s, at 72 °C for 1min (wherein including 35 cycles at 95°C for 30s, at 60°C for 30s, and at 72°C for 1min, respectively), and at 72°C for 10min.
3) After the reaction, the above reaction products are electrophorized in 1% agarose gel at 100V for 40min.
4) After electrophoresis, a target band (as shown in FIG. 9, 1kb shows a 1kb DNA marker, a first lane is the corresponding double-stranded DNA, a second lane is the reverse product amplification result, and a third lane is the forward product amplification result), and recovering DNA using a gel cutting recovery kit.
5) EGFP lssDNA is analyzed quantitatively using nanodrop.

### 6.2 Expression effect of linear single-stranded DNA

1) 1,5pg of cationic polymer (Lipofectamine 2000) is added into 50µL of MEM medium, mixed well at room temperature and placed for 5min. Subsequently, 0.5µg of EGFP lssDNA (in 50µL MEM medium) is mixed with 1.5µg of the prepared cationic polymer (Lipofectamine 2000, already in 50µL MEM medium) to obtain 100µL of transfection composition, and stored at room temperature for 15 min.
2) The above mixed solution is added dropwise to 293T cells and MGC803 cells that are laid on a 24 well plate in advance, and cultured for 24h.
3) The expression of EGFP lssDNA is observed by using a fluorescence microscope (the results are shown in FIG. 10).

As shown in FIG. 10 that linear single-stranded DNA can also express the target protein product in cells after transfection.

### Example 7. Comparison of immune response in vitro between single-stranded DNA and double-stranded plasmid DNA after transfection

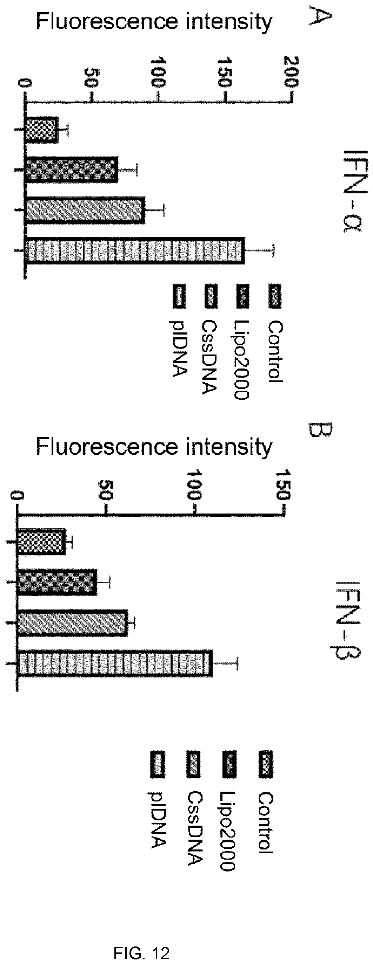

The experimental steps are briefly described as follows:
1) preparing a macrophage suspension and counting cells;
2) inoculating macrophages into a 6-well plate: the plate is laid with a density of 5×10⁵ cells/well, with about 2mL of cell suspension in each well;
3) culturing in an incubator at 37°C for 12-24h;
4) adding respectively a composition of Lipofectamine 2000 (2µg), EGFP cssDNA (0.6µg) and transfection reagent Lipofectamine 2000 (2µg), and a composition of double-stranded plasmid DNA (1.2µg) and transfection reagent Lipofectamine 2000 (2µg);
5) incubating in the incubator at 37°C for 24h;
6) collecting a cell suspension; and
7) co-incubating an FITC fluorescent labeled IFN-α antibody (Thermo Fisher Scientific, Article No. PA5-115430) and an IFN-β antibody (Thermo Fisher Scientific, Article No. PA5-95722) with each group of cell suspensions; and
8) detecting the fluorescence intensity of each group by flow cytometry, and then performing data analysis and processing, wherein the results are as shown in FIG.12A (IFN-α) and FIG.12B (IFN-β).

The results of this example show that the single-stranded DNA has lower immune stimulation to macrophages at a cellular level compared with the corresponding double-stranded plasmid DNA.

### Example 8. Comparison of immune response in vivo between single-stranded DNA and double-stranded plasmid DNA after transfection

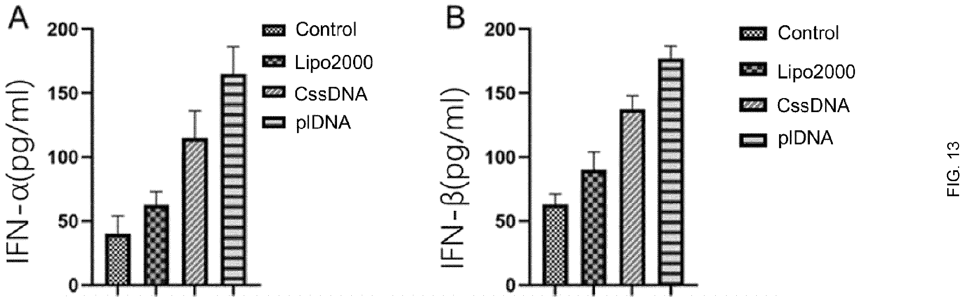

The experimental steps are briefly described as follows:
1) twenty female mice are divided randomly into four groups, five in each group, which are a control group, a Lipofectamine 2000 administration group, an EGFP cssDNA and Lipofectamine 2000 complex administration group and a double-stranded plasmid DNA and Lipofectamine 2000 complex administration group.
2) A composition of EGFP cssDNA (9µg) and transfection reagent Lipofectamine 2000 (100µL) is prepared, and a composition of double-stranded plasmid DNA (18µg) and transfection reagent Lipofectamine 2000 (100µL) is prepared.
3) Different groups are administrated by tail vein injection respectively. The control group is administrated with 100µL of normal saline, the liposome group is administrated with the same mass of 100µL Lipofectamine 2000 solution, the single-stranded DNA administration group is administrated with 9µg of EGFP cssDNA each by tail vein injection, and the plDNA administration group is administrated with 18µg plDNA each by tail vein injection.
4) One week later, the peripheral blood of each group is extracted and treated according to the instructions of Elisa kits (Abcam, Article No. ab252352 and No. ab252363) to determine the concentration of type I interferons (IFN-α and IFN-β) in the blood. The results are shown in FIG. 13A (IFN-α) and FIG. 13B (IFN-β).

The results of this example show that compared with the corresponding double-stranded plasmid plDNA, single-stranded DNA has lower immune stimulation *in vivo,* indicating that it has lower side effects and better safety in clinical practice, and has greater clinical application value.

Although the above examples provide a detailed description of the present invention, they are only a part of examples, not all embodiments of the present invention. A person may also obtain other examples without inventive premise according to the present examples, and these examples are within the protection scope of the present invention.

## Claims

1. A composition, comprising single-stranded DNA and at least one transfection reagent, wherein a value of a mass ratio of the single-stranded DNA to the transfection reagent in the composition is about 100 to about 0.1.

2. The composition according to claim 1, wherein the mass ratio of the single-stranded DNA to the transfection reagent is about 100:1 to about 1:10.

3. The composition according to any one of claims 1 to 2, wherein the value of the mass ratio of the single-stranded DNA to the transfection reagent is about 1 to about 0.2.

4. The composition according to any one of claims 1 to 3, wherein the mass ratio of the single-stranded DNA and the transfection reagent is about 2:1 to about 1:5.

5. The composition according to any one of claims 1 to 4, wherein the single-stranded DNA is circular single-stranded DNA.

6. The composition according to any one of claims 1 to 4, wherein the single-stranded DNA is linear single-stranded DNA.

7. The composition according to any one of claims 1 to 6, wherein the single-stranded DNA comprises a nucleic acid sequence encoding one or more RNAs and/or proteins.

8. The composition according to any one of claims 1 to 7, wherein the single-stranded DNA comprises a nucleic acid sequence encoding two or more RNAs and/or proteins.

9. The composition according to any one of claims 1 to 8, wherein the single-stranded DNA comprises a sense strand nucleic acid sequence encoding RNA and/or protein.

10. The composition according to any one of claims 1 to 9, wherein the single-stranded DNA comprises an antisense strand nucleic acid sequence encoding RNA and/or protein.

11. The composition according to any one of claims 1 to 10, wherein the single-stranded DNA comprises at least 40 nucleotides.

12. The composition according to any one of claims 1 to 11, wherein the single-stranded DNA comprises at least 1500 nucleotides.

13. The composition according to any one of claims 1 to 12, wherein the transfection reagent comprises a cationic lipid and/or a cationic polymer.

14. The composition according to any one of claims 1 to 13, wherein the transfection reagent comprises a cationic liposome.

15. The composition according to any one of claims 1 to 14, further comprising one or more cell cultures.

16. The composition according to any one of claims 1 to 15, wherein the content of the single-stranded DNA is about 10ng to about 1000ng.

17. The composition according to any one of claims 1 to 16, wherein a concentration of the single-stranded DNA is about 0.5nM to about 3nM.

18. A kit, comprising the composition according to any one of claims 1 to 17.

19. A kit, comprising single-stranded DNA; at least one transfection reagent; and instructions for use, wherein the instructions for use record that the single-stranded DNA and the transfection reagent are applied to target cells in a mass ratio of about 100 to about 0.1 to deliver at least part of the nucleic acid contained in the single-stranded DNA to the target cells.

20. The kit according to claim 19, wherein the instructions for use record that the single-stranded DNA and the transfection reagent are applied to the target cells in a mass ratio of about 5:1 to about 1:10 to deliver at least part of the nucleic acid contained in the single-stranded DNA to the target cells.

21. The kit according to any one of claims 19 to 20, wherein the instructions for use record that the single-stranded DNA and the transfection reagent are applied to the target cells in a mass ratio of about 10 to about 0.1 to deliver at least part of the nucleic acid contained in the single-stranded DNA to the target cells.

22. The kit according to any one of claims 19 to 21, wherein the instructions for use record that the single-stranded DNA and the transfection reagent are applied to the target cells in a mass ratio of about 2:1 to about 1:5 to deliver at least part of the nucleic acid contained in the single-stranded DNA to the target cells.

23. The kit according to any one of claims 19 to 22, wherein the instructions for use record that the single-stranded DNA and the transfection reagent are mixed according to said mass ratio to prepare a transfection composition.

24. The kit according to any one of claims 19 to 23, wherein the single-stranded DNA and the at least one transfection reagent are included in the kit in a manner of not mixing with each other.

25. The kit according to any one of claims 19 to 24, wherein the single-stranded DNA and the at least one transfection reagent are separately stored in a separately packaged container.

26. The kit according to any one of claims 19 to 25, wherein the content of the single-stranded DNA is about 10ng to about 1000ng.

27. The kit according to any one of claims 19 to 26, wherein the concentration of the single-stranded DNA is about 0.5nM to about 3nM.

28. The kit according to any one of claims 19 to 27, wherein the single-stranded DNA is circular single-stranded DNA.

29. The kit according to any one of claims 19 to 27, wherein the single-stranded DNA is linear single-stranded DNA.

30. The kit according to any one of claims 19 to 29, wherein the single-stranded DNA comprises a nucleic acid sequence encoding one or more RNAs and/or proteins.

31. The kit according to any one of claims 19 to 30, wherein the single-stranded DNA comprises a nucleic acid sequence encoding two or more RNAs and/or proteins.

32. The kit according to any one of claims 19 to 31, wherein the single-stranded DNA comprises a sense stranded nucleic acid sequence encoding RNA and/or protein.

33. The kit according to any one of claims 19 to 32, wherein the single-stranded DNA comprises an antisense stranded nucleic acid sequence encoding RNA and/or protein.

34. The kit according to any one of claims 19 to 33, wherein the single-stranded DNA comprises at least 40 nucleotides.

35. The kit according to any one of claims 19 to 34, wherein the single-stranded DNA comprises at least 1500 nucleotides.

36. The kit according to any one of claims 19 to 35, wherein the transfection reagent comprises a cationic lipid and/or cationic polymer.

37. The kit according to any one of claims 19 to 36, wherein the transfection reagent comprises a cationic liposome.

38. The kit according to any one of claims 19 to 37, further comprising one or more cell culture mediums.

39. A method for delivering a nucleic acid to cells, comprising: contacting the cells with a transfection reagent and single-stranded DNA containing the nucleic acid, wherein a mass ratio of the single-stranded DNA to the transfection reagent is about 5 to about 0.1.

40. The method according to claim 39, wherein the mass ratio of the single-stranded DNA to the transfection reagent is about 5:1 to about 1:10.

41. The method according to any one of claims 39 to 40, wherein the mass ratio of the single-stranded DNA to the transfection reagent is about 1 to about 0.2.

42. The method according to any one of claims 39 to 41, wherein the mass ratio of the single-stranded DNA to the transfection reagent is about 2:1 to about 1:5.

43. A method for expressing foreign RNA and/or foreign protein in cells, comprising: introducing single-stranded DNA encoding the foreign RNA and/or the foreign protein into the cells.

44. The method according to claim 43, further comprising: growing the cells under a condition that allows the expression of the foreign RNA and/or the foreign protein.

45. A method for reducing the cytotoxicity related to DNA transfection, comprising: introducing single-stranded DNA rather than corresponding double-stranded DNA into the cells during transfection.

46. A method for reducing a cellular immune response related to DNA transfection, comprising: introducing single-stranded DNA rather than corresponding double-stranded DNA into cells during transfection.

47. A method for improving the DNA transfection efficiency of cells, comprising: introducing single-stranded DNA rather than corresponding double-stranded DNA into the cells during transfection.

48. The method according to any one of claims 43 to 47, wherein the introduction of the single-stranded DNA into the cells comprises: contacting the cells with the composition according to any one of claims 1 to 17.

49. The method according to any one of claims 43 to 47, wherein the introduction of the single-stranded DNA into the cells comprises: contacting the cells with the single-stranded DNA and a transfection reagent, wherein a mass ratio of the single-stranded DNA to the transfection reagent is about 5 to about 0.1.

50. The method according to any one of claims 43 to 47 and 49, wherein the introduction of the single-stranded DNA into the cells comprises: contacting the cells with the single-stranded DNA and the transfection reagent, wherein the mass ratio of the single-stranded DNA to the transfection reagent is about 5:1 to about 1:10.

51. The method according to any one of claims 43 to 47 and 49 to 50, wherein the introduction of the single-stranded DNA into the cells comprises: contacting the cells with the single-stranded DNA and the transfection reagent, wherein the mass ratio of the single-stranded DNA to the transfection reagent is about 1 to about 0.2.

52. The method according to any one of claims 43 to 47 and 49 to 51, wherein the introduction of the single-stranded DNA into the cells comprises: contacting the cells with the single-stranded DNA and the transfection reagent, wherein the mass ratio of the single-stranded DNA to the transfection reagent is about 2:1 to about 1:5.

53. The method according to any one of claims 39 and 48 to 52, wherein the contact comprises: incubating at about 25°C to about 40°C.

54. The method according to any one of claims 39 and 48 to 53, wherein the contact comprises: incubating at about 37°C.

55. The method according to any one of claims 39 and 48 to 54, wherein the contact comprises: incubating for at least 4 hours.

56. The method according to any one of claims 39 to 55, wherein the single-stranded DNA is circular single-stranded DNA.

57. The method according to any one of claims 39 to 56, wherein the single-stranded DNA is linear single-stranded DNA.

58. The method according to any one of claims 39 to 57, wherein the single-stranded DNA comprises a nucleic acid sequence encoding one or more foreign RNAs and/or foreign proteins.

59. The method according to any one of claims 39 to 58, wherein the single-stranded DNA comprises a nucleic acid sequence encoding two or more foreign RNAs and/or foreign proteins.

60. The method according to any one of claims 39 to 59, wherein the single-stranded DNA comprises a sense stranded nucleic acid sequence encoding foreign RNA and/or foreign protein.

61. The method according to any one of claims 39 to 60, wherein the single-stranded DNA comprises an antisense stranded nucleic acid sequence encoding foreign RNA and/or foreign protein.

62. The method according to any one of claims 39 to 61, wherein the single-stranded DNA comprises at least 40 nucleotides.

63. The method according to any one of claims 39 to 62, wherein the single-stranded DNA comprises at least 1500 nucleotides.

64. The method according to any one of claims 39 to 63, wherein the content of the single-stranded DNA is about 10ng to about 1000ng.

65. The method according to any one of claims 39 to 64, wherein a concentration of the single-stranded DNA is about 0.5nM to about 3nM.

66. The method according to any one of claims 39 to 42 and 49 to 65, wherein the transfection reagent comprises a cationic lipid and/or cationic polymer.

67. The method according to any one of claims 39 to 42 and 49 to 66, wherein the transfection reagent comprises a cationic liposome.

68. The method according to any one of claims 39 to 67, wherein the method is an *in vivo* method, an *in vitro* method or an .

69. The method according to any one of claims 39 to 68, wherein the cells are somatic cells, tumor cells and/or immune cells.

70. The method according to any one of claims 39 to 69, wherein the cells are selected from: lung cancer cells, kidney cells, melanoma cells, liver cancer cells, cervical cancer cells, glioma cells, epithelial cells, colon cancer cells, fibroblasts, breast cancer cells, stomach cancer cells and immune effector cells.

71. The method according to any one of claims 39 to 70, wherein the cells are selected from A549 cells, C6 cells, MCF-7 cells, HepG2 cells, CT26 cells, Hela cells, Helf cells, MDCK cells, B16 cells, Hep3B cells, L929 cells, 4T1 cells, 293T cells, MGC803 cells and macrophages.

72. The cells obtained by the method of any one of claims 39 to 71.

73. An organism or components thereof, comprising the cells according to claim 72.

74. The organism or components thereof according to claim 73, wherein the organism is a non-human organism.

75. The organism or components thereof according to any one of claims 73 to 74, wherein the components are selected from tissues, organs and combinations thereof.

76. Use of single-stranded DNA in the preparation of a transfection composition, the transfection being used for one or more of the followings: delivering at least part of nucleic acid contained in the single-stranded DNA to cells; expressing foreign RNA or foreign protein encoded by the single-stranded DNA in the cells; improving the DNA transfection efficiency of the cells; reducing the cytotoxicity related to DNA transfection; and/or reducing a cellular immune response related to DNA transfection.

77. The use according to claim 76, wherein the transfection composition is the composition according to any one of claims 1 to 17.

78. The use according to any one of claims 76 to 77, wherein the cells are somatic cells, tumor cells and/or immune cells.

79. The use according to any one of claims 76 to 78, wherein the cells are selected from: lung cancer cells, kidney cells, melanoma cells, liver cancer cells, cervical cancer cells, glioma cells, epithelial cells, colon cancer cells, fibroblasts, breast cancer cells, stomach cancer cells and immune effector cells.

80. The use according to any one of claims 76 to 79, wherein the cells are selected from: A549 cells, C6 cells, MCF-7 cells, HepG2 cells, CT26 cells, Hela cells, Helf cells, MDCK cells, B16 cells, Hep3B cells, L929 cells, 4T1 cells, 293T cells, MGC803 cells and macrophages.
